# FASCICULE DE BREVET EUROPEEN

(11) **EP 1 509 520 B1**
(45) Date de publication et mention de la délivrance du brevet: **22.11.2006**
(21) Numéro de dépôt: 03757110.6
(22) Date de dépôt: 05.06.2003
(51) Int. Cl.: C07D 455/06, C07D 471/14

(54) **UTILISATION DE DERIVES DES BENZO(C)QUINOLIZINIUMS POUR LE TRAITEMENT DES PATHOLOGIES LIEES AUX PHENOMENES DE CONSTRICTION DES CELLULES MUSCULAIRES LISSES**
VERWENDUNG VON BENZO(C)CHINOLIZINIUMDERIVATEN ZUR BEHANDLUNG VON KRANKHEITEN DIE DURCH VERENGUNGEN DER GLATTEN MUSKELZELLEN BEDINGT SIND
USE OF BENZO(C)QUINOLIZINIUM DERIVATIVES FOR THE TREATMENT OF DISEASES THAT ARE LINKED TO SMOOTH MUSCLE CELL CONSTRICTION

(30) Priorité: 05.06.2002 FR 0206916
(43) Date de publication de la demande: 02.03.2005
(73) Titulaire: CENTRE NATIONAL DE LA RECHERCHE SCIENTIFIQUE (CNRS), 75794 Paris Cedex 16 (FR); UNIVERSITE DE POITIERS, 86034 Poitiers Cedex (FR)
(72) Inventeur: BECQ, Frédéric, F-86280 St Benoît (FR); ROBERT, Renaud, F-79400 AUGE (FR); PIGNOUX, Laurence, F-86800 Jardres (FR); ROGIER, Christian, F-86000 Poitiers (FR); METTEY, Yvette, F-86000 Poitiers (FR); VIERFOND, Jean, Michel, F-94700 Maisons Alfort (FR); JOFFRE, Michel, F-31300 Toulouse (FR); MARIVINGT-MOUNIR, Cécile, F-86000 Poitiers (FR)
(74) Mandataire: Grosset-Fournier, Chantal Catherine
(86) Numéro de dépôt international: PCT/FR2003/001688
(87) Numéro de publication internationale: WO 2003/104228

(56) Documents cités:
- WO-A-98/05642

## Description

La présente invention a pour objet l'utilisation de dérivés des benzo[c]quinoliziniums dans le cadre du traitement de pathologies liées aux phénomènes de constriction des cellules musculaires lisses, telles que l'hypertension artérielle, ou l'asthme.

De nombreux activateurs moléculaires ont été synthétisés en vue de leur utilisation éventuelle dans le cadre du traitement de la mucoviscidose (Becq *et al.,* 1999; WO 98/05642). De ces synthèses chimiques sont apparues une série de composés substitués du benzo[c]quinolizinium (MPB) et, parmi ces molécules, les composés MPB-07 et MPB-91. Dans des cellules exprimant la protéine CFTR de manière endogène, il a été démontré, par des techniques d'efflux iodure et de patch clamp, que les MPB-07 et MPB-91 sont des activateurs de ce canal ionique (Becq *et al.,* 1999, Dérand et al., 2001). Les inventeurs ont montré que ces deux molécules modifient la localisation de deIF508-CFTR dans les cellules pulmonaires de patients homozygotes pour cette mutation, et qu'une activation du canal delF508 est restaurée après traitement (Dormer *et al.,* 2001). Le mécanisme d'action n'est pas établi mais ces deux molécules n'affectent pas l'activité ATPase des deux sites NBF (Dérand *et al.,* 2001), ni le taux d'AMPc intracellulaire ni les activités phosphatases et kinases des principales enzymes impliquées dans la régulation de CFTR (Becq et al., 1999, Dérand et al., 2001).

La présente invention découle de la mise en évidence par les inventeurs des effets du MPB-07 et du MPB-91 sur la réactivité vasculaire. Les inventeurs ont utilisé l'aorte de rat, organe ayant la propriété de se contracter (vasoconstriction) ou de se relaxer (vasorelaxation) sous l'action de différents agents moléculaires (endogènes ou exogènes) ou mécaniques.

Ainsi les inventeurs ont démontré que les benzo[c]quinoliziniums décrits ci-après dans le cadre de la présente invention, tels que le MPB-07 et le MPB-91, sont des molécules capables de moduler le tonus vasculaire chez l'homme et chez le rat en agissant sur l'activité des cellules musculaires lisses. De plus, les inventeurs ont démontré que CFTR est exprimé et fonctionnel dans le muscle lisse. Ces résultats démontrent qu'une molécule qui se révèlerait être activatrice de CFTR, peut agir comme un vasorelaxant et un broncho-dilatateur potentiel.

A ce titre, la présente invention a pour but de fournir de nouveaux médicaments destinés au traitement de pathologies liées à une constriction des cellules musculaires lisses dans les tissus telles que les pathologies liées aux phénomènes de vasoconstriction dans le cadre de troubles vasculaires, notamment l'hypertension artérielle, ou les pathologies liées aux phénomènes de bronchoconstriction dans le cadre de troubles de la respiration, notamment l'asthme.

La présente invention a pour objet l'utilisation de composés activateurs du canal CFTR, tels que les benzo[c]quinoliziniums, pour la préparation de médicaments destinés au traitement de pathologies liées à une constriction des cellules musculaires lisses dans les tissus telles que les pathologies liées aux phénomènes de vasoconstriction dans le cadre de troubles vasculaires, notamment l'hypertension artérielle, ou les pathologies liées aux phénomènes de bronchoconstriction dans le cadre de troubles de la respiration, notamment l'asthme.

La présente invention a plus particulièrement pour objet l'utilisation de dérivés de formule générale (I) suivante : 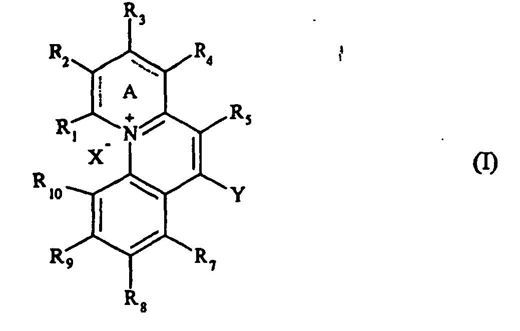 dans laquelle :
- l'hétérocycle A est aromatique ou non, étant entendu que dans ce dernier cas l'atome d'azote de cet hétérocycle est lié par une double liaison au carbone en position 4a,
- R₁, R₂, R₃, R₄, R₅, R₇, R₈, R₉ et R₁₀, représentent, indépendamment les uns des autres :
   - un atome d'hydrogène, ou
   - un atome d'halogène, notamment un atome de chlore, de brome, ou de fluor, ou
   - un groupe alkyle, alkoxy, carbonyle, oxycarbonyle, ou ester, linéaire ou ramifié, d'environ 1 à environ 10 atomes de carbone, ces groupes étant le cas échéant substitués, notamment par un halogène, et/ou par un hydroxyle, et/ou par une amine (primaire, secondaire ou tertiaire), et/ou par un cycle aromatique et/ou aliphatique, d'environ 5 à environ 10 atomes de carbone dans le cycle, ces cycles étant eux-mêmes, le cas échéant, substitués notamment par un halogène, et/ou par un hydroxyle, et/ou par une amine (primaire, secondaire ou tertiaire), et/ou par un groupe alkyle, alkoxy, carbonyle, oxycarbonyle, ou ester, ces groupes étant tels que définis ci-dessus, ou
   - un cycle aromatique ou aliphatique, d'environ 5 à environ 10 atomes de carbone dans le cycle, ce cycle étant lui-même, le cas échéant, substitué notamment par un halogène, et/ou par un hydroxyle, et/ou par une amine (primaire, secondaire ou tertiaire), et/ou par un groupe alkyle, alkoxy, carbonyle, oxycarbonyle, ou ester, ces groupes étant tels que définis ci-dessus, ou
   - un groupe -ORₐ, Rₐ représentant un atome d'hydrogène, ou un groupe alkyle, carbonyle, oxycarbonyle, ou ester, linéaire ou ramifié, ces groupes étant tels que définis ci-dessus, ou un cycle aromatique ou aliphatique, ces cycles étant tels que définis ci-dessus, ou
   - un groupe -NR_{b}R_{c}, R_{b} et R_{c}, indépendamment l'un de l'autre, représentant un atome d'hydrogène, ou un groupe alkyle, alkoxy, carbonyle, oxycarbonyle, ou ester, linéaire ou ramifié, ces groupes étant tels que définis ci-dessus, ou un cycle aromatique ou aliphatique, ces cycles étant tels que définis ci-dessus, ou
   - lorsque R₁ et R₂, et/ou R₃ et R₄, et/ou R₄ et R₅, et/ou R₇ et R₈, et/ou R₈ et R₉, et/ou R₉ et R₁₀, ne représentent pas les différents atomes ou groupes ou cycles mentionnés ci-dessus, alors R₁ en association avec R₂; et/ou R₂ en association avec R₃, et/ou R₃ en association avec R₄, et/ou R₄ en association avec R₅, et/ou R₇ en association avec R₈, et/ou R₈ en association avec R₉, et/ou R₉ en association avec R₁₀, forment respectivement avec C₁ et C₂, ou avec C₂ et C₃, ou avec C₃ et C₄, ou avec C₄, C₄ₐ et C₅, ou avec C₇ et C₈, ou avec C₈ et C₉, ou avec C₉ et C₁₀, un cycle aromatique ou aliphatique de 5 à 10 atomes de carbone, ce cycle étant le cas échéant substitué, notamment par un halogène, et/ou par un groupe alkyle, alkoxy, carbonyle, oxycarbonyle, ou ester, et/ou par un cycle aromatique ou aliphatique, ces groupes ou cycles étant tels que définis ci-dessus, ou
   - lorsque R₃ et R₄ ne représentent pas les différents atomes ou groupes ou cycles mentionnés ci-dessus, alors R₃ en association avec R₄ forment un groupe indole de formule 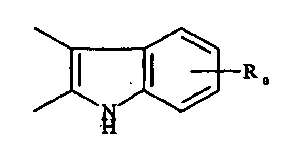
dans laquelle Rₐ est tel que défini ci-dessus,
- Y représente :
   - un groupe -OR_{d}, R_{d} représentant un atome d'hydrogène, ou un groupe alkyle, carbonyle, oxycarbonyle, ou ester, linéaire ou ramifié, ces groupes étant tels que définis ci-dessus, ou un cycle aromatique ou aliphatique, ces cycles étant tels que définis ci-dessus, ou
   - un groupe -NRₑR_{f}, Rₑ et R_{f} indépendamment l'un de l'autre, représentant un atome d'hydrogène, ou un groupe alkyle, alkoxy, carbonyle, oxycarbonyle, ou ester, linéaire ou ramifié, ces groupes étant tels que définis ci-dessus, ou un cycle aromatique ou aliphatique, ces cycles étant tels que définis ci-dessus,
   - ou un groupe SH,
   - étant entendu que lorsque R_{d}, ou l'un au moins de Rₑ ou de R_{f}, ne représentent pas l'un des différents atomes ou groupes ou cycles mentionnés ci-dessus, alors R_{d}, ou l'un au moins de Rₑ ou de R_{f}, en association avec R₅, ou en association avec R₇, forment respectivement avec C₅ et C₆, ou avec C₆, C₆ₐ et C₇, un hétérocycle aromatique ou aliphatique de 5 à 10 atomes de carbone, le cas échéant substitué, notamment par un halogène, et/ou par un groupe alkyle, alkoxy, carbonyle, oxycarbonyle, ou ester, et/ou par un cycle aromatique ou aliphatique, ces groupes ou cycles étant tels que définis ci-dessus,
- X représente un atome sous forme anionique, tel qu'un atome d'halogène, notamment un atome de brome ou de chlore, ou un groupe d'atomes sous forme anionique, tel qu'un perchlorate, et l'azote de l'hétérocycle A de la formule (I) est sous forme quaternaire et est lié d'une part par liaison covalente au carbone en position 11, et, d'autre part, par liaison ionique à X défini ci-dessus, étant entendu que lorsque R₁ et R₁₀ ne représentent pas l'un des différents atomes ou groupes ou cycles mentionnés ci-dessus, alors R₁ en association avec R₁₀ forment avec C₁, l'azote de l'hétérocycle A de la formule (I), C₁₁, et C₁₀, un hétérocycle aromatique ou aliphatique de 5 à 10 atomes de carbone, le cas échéant substitué, notamment par un halogène, et/ou par un groupe alkyle, alkoxy, carbonyle, oxycarbonyle, ou ester, et/ou par un cycle aromatique ou aliphatique, ces groupes ou cycles étant tels que définis ci-dessus,
pour la préparation de médicaments destinés au traitement de pathologies liées à une constriction des cellules musculaires lisses dans les tissus telles que les pathologies liées aux phénomènes de vasoconstriction dans le cadre de troubles vasculaires, notamment l'hypertension artérielle, ou les pathologies liées aux phénomènes de bronchoconstriction dans le cadre de troubles de la respiration, notamment l'asthme.

L'invention a plus particulièrement pour objet l'utilisation telle que décrite ci-dessus, des dérivés des benzo[c] quinoliziniums de formule (Ia) suivante : 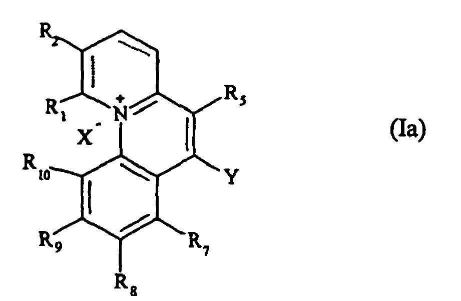 dans laquelle :
- R₁ et R₂ représentent un atome d'hydrogène, ou forment en association avec C₁ et C₂ un cycle aromatique à 6 atomes de carbone,
- R₅ représente un atome d'hydrogène, ou un groupe alkyle linéaire ou substitué de 1 à 10 atomes de carbone, notamment un groupe butyle, ou un ester de formule COOR' dans laquelle R' représente un groupe alkyle linéaire ou substitué de 1 à 10 atomes de carbone, notamment un groupe éthyle,
- Y représente un groupe -OH, -SH, -NH₂, ou -NHCOCH₃,
- R₇, R₈, R₉ et R₁₀ représentent un atome d'hydrogène, ou l'un au moins de R₇, R₈, R₉ ou R₁₀, représente un atome d'halogène, notamment un atome de chlore de brome ou de fluor,
- X représente un atome d'halogène sous forme anionique, notamment un atome de brome Br⁻, ou de chlore Cl⁻, ou un groupe d'atomes sous forme anionique.

L'invention a également plus particulièrement pour objet des dérivés des benzo[c] quinoliziniums de formule (Ia) tels que définis ci-dessus, dans laquelle Y représente un groupe -NH₂, ou -NHCOCH₃.

A ce titre, l'invention concerne plus particulièrement encore l'utilisation telle que décrite ci-dessus, des dérivés des benzo[c]quinoliziniums de formule (Ia) suivants :

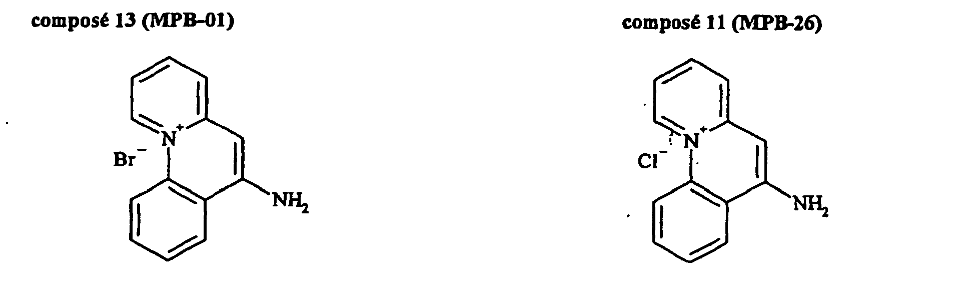

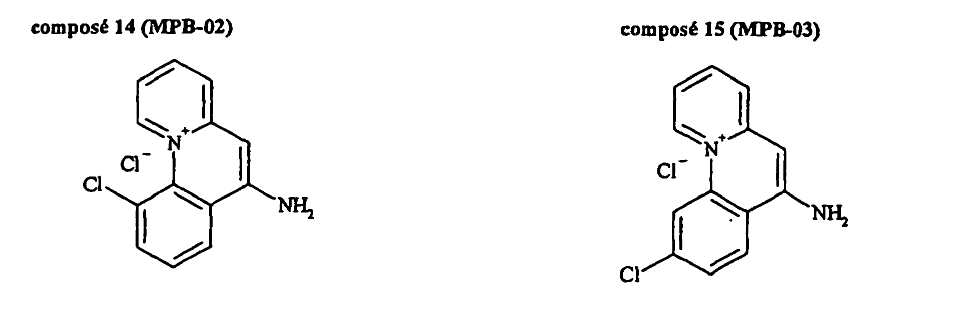

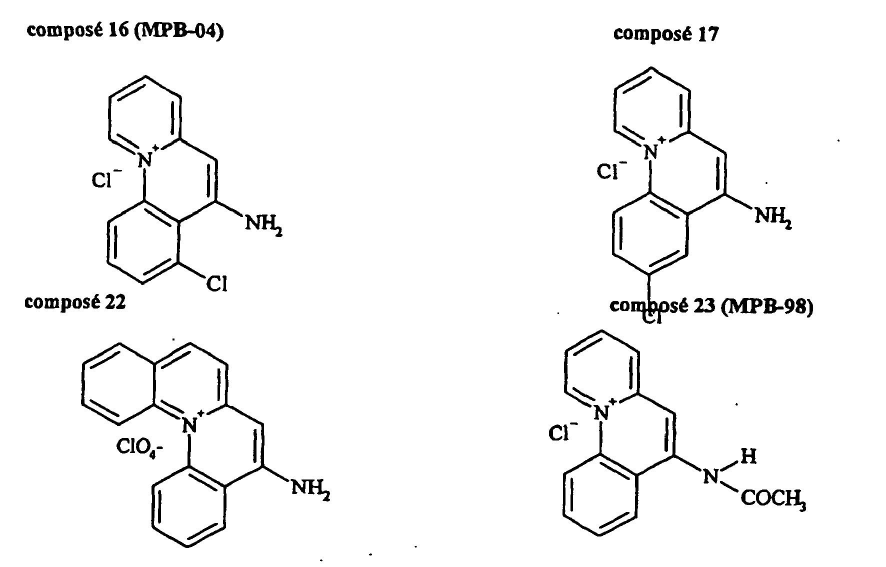

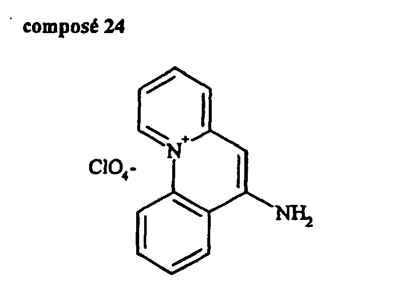

L'invention a également plus particulièrement pour objet l'utilisation telle que décrite ci-dessus, des dérivés des benzo[c] quinoliziniums de formule (Ia) tels que définis ci-dessus, dans laquelle Y représente OH.

A ce titre, l'invention concerne plus particulièrement encore l'utilisation telle que décrite ci-dessus, des dérivés des benzo[c]quinoliziniums de formule (Ia) suivants :

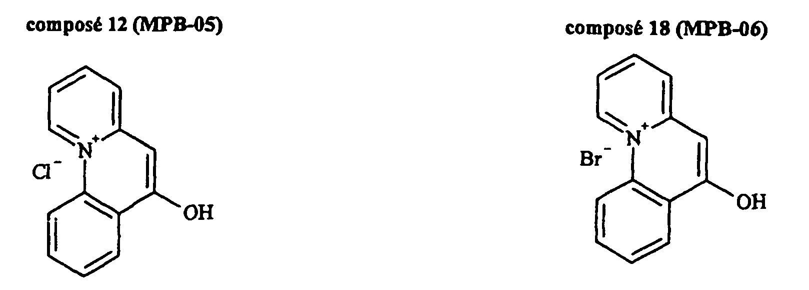

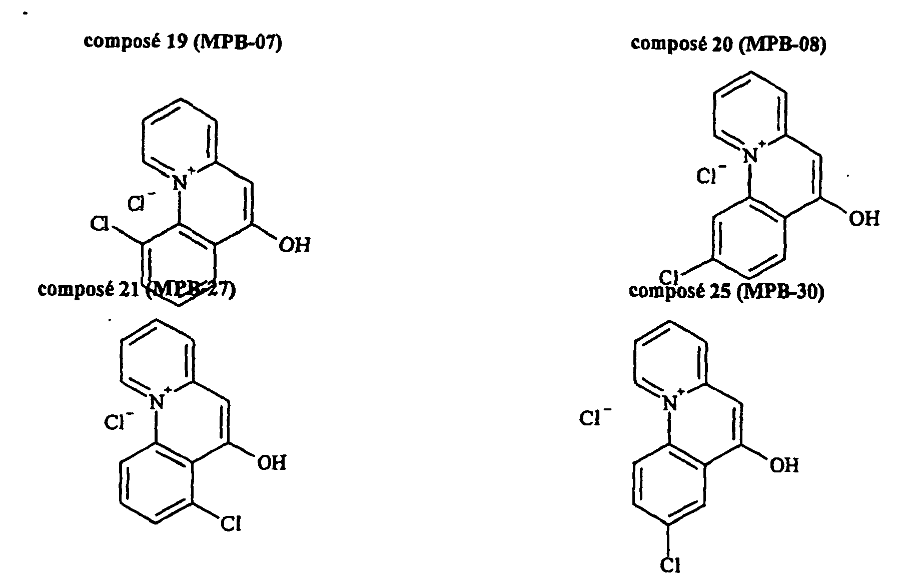

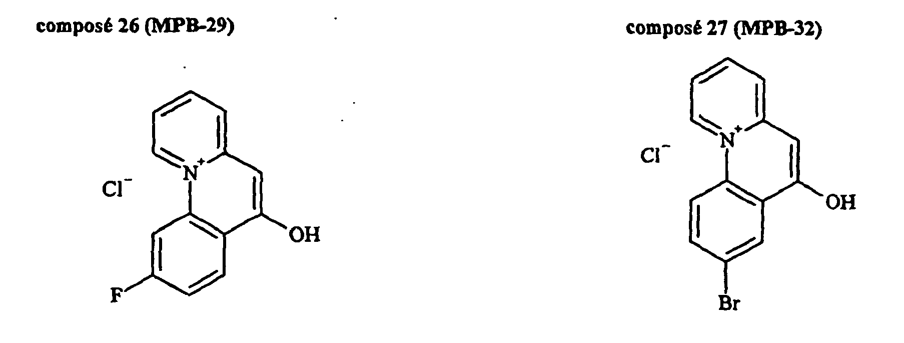

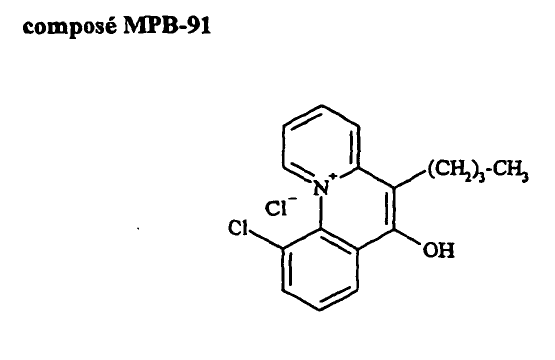

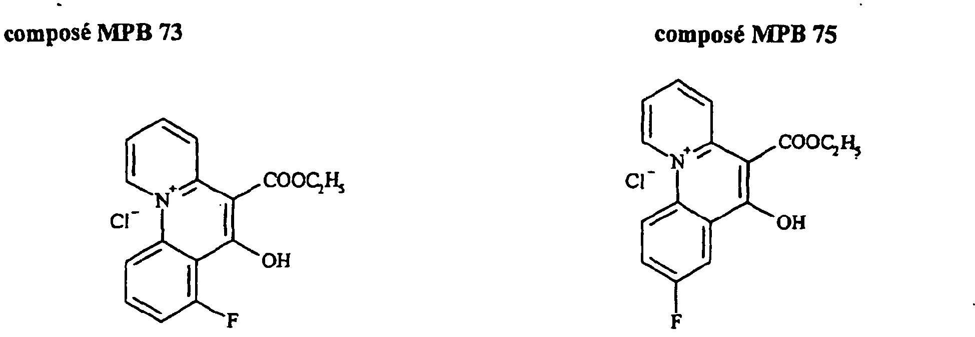

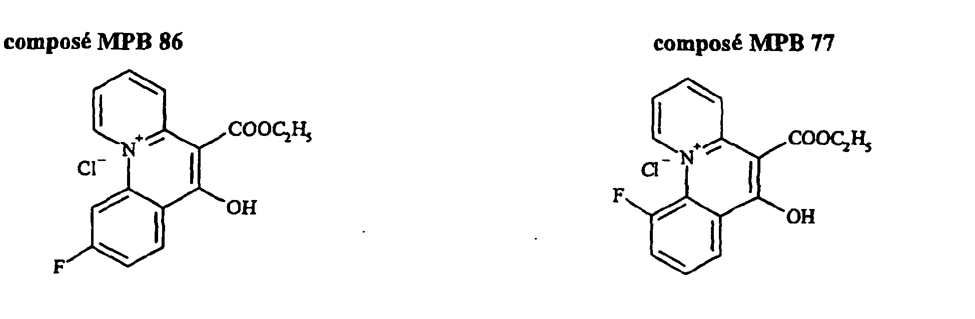

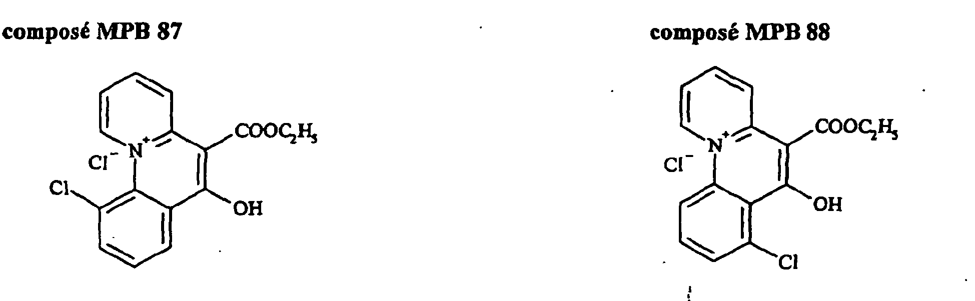

L'invention a également plus particulièrement pour objet l'utilisation telle que décrite ci-dessus, des dérivés des benzo[c] quinoliziniums de formule (Ia) tels que définis ci-dessus, dans laquelle Y représente SH.

A ce titre, l'invention concerne plus particulièrement encore l'utilisation telle que décrite ci-dessus, des dérivés des benzo[c]quinoliziniums de formule (Ia) suivants :

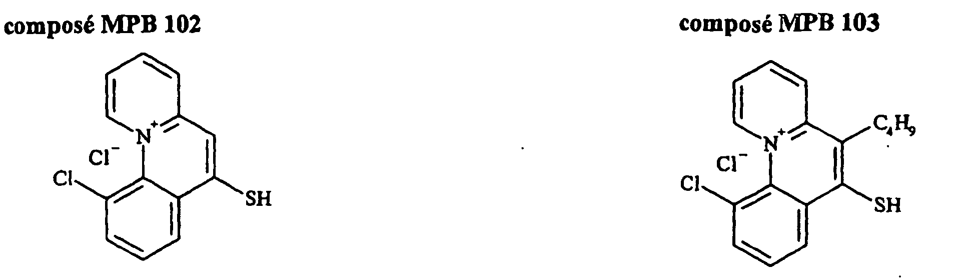

L'invention concerne également l'utilisation telle que décrite ci-dessus de dérivés de formule générale (Ia-1) suivante : 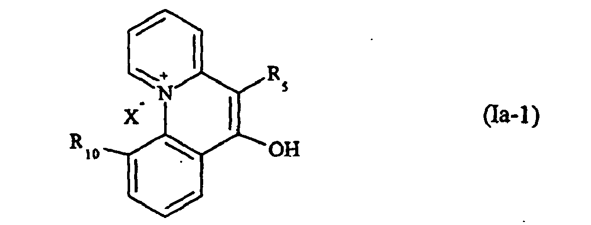 dans laquelle :
- R₅ représente un atome d'hydrogène, ou un groupe alkyle linéaire ou substitué de 1 à 10 atomes de carbone, notamment un groupe butyle,
- R₁₀ représente un atome un atome d'halogène, notamment un atome de chlore, de brome ou de fluor,
- X représente un atome d'halogène sous forme anionique, notamment un atome de brome Br, ou de chlore Cl⁻, ou un groupe d'atomes sous forme anionique.

L'invention a plus particulièrement pour objet l'utilisation susmentionnée du dérivé de formule (Ia-1) définie ci-dessus, et correspondant au composé MPB-07 de formule suivante :

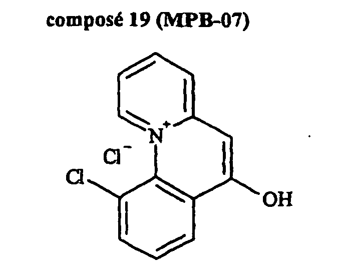

L'invention concerne plus particulièrement également l'utilisation susmentionnée du dérivé de formule (Ia-1) définie ci-dessus, et correspondant au composé MPB-91 de formule suivante :

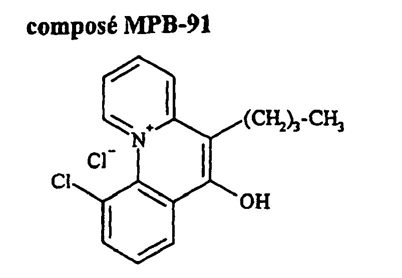

L'invention a également pour objet l'utilisation susmentionnée de dérivés de formule générale (Ib) suivante : 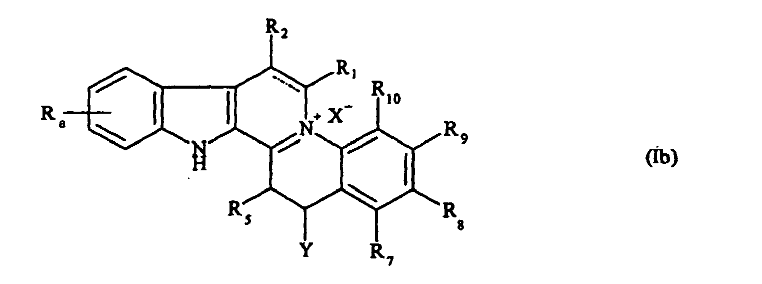 dans laquelle Rₐ, R₁, R₂, R₅, R₇, R₈, R₉, R₁₀, X et Y sont tels que définis ci-dessus, et notamment les composés de formule (Ib) dans laquelle :
- Rₐ représente un atome d'hydrogène,
- R₁ et R₂ représentent un atome d'hydrogène, et il n'y a pas de double liaison entre les deux carbones portant R₁ et R₂,
- R₅ représente un atome d'hydrogène,
- R₇, R₈, R₉ et R₁₀ représentent un atome d'hydrogène, ou l'un de R₇, R₈, R₉ ou R₁₀ représente un atome d'halogène, notamment un atome de chlore, de brome ou de fluor,
- Y représente NH₂, ou OH,
- X représente un atome d'halogène, notamment un atome de brome, ou de chlore, ou de fluor.

L'invention a plus particulièrement pour objet l'utilisation susmentionnée de dérivés de formule (Ib-1) suivante : 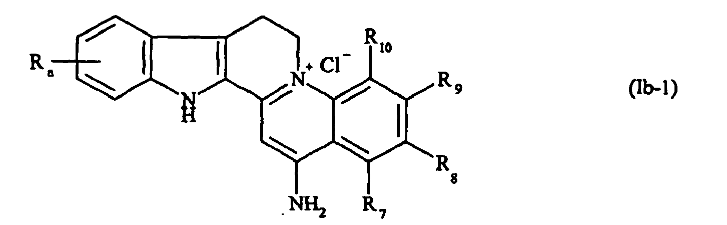 dans laquelle Rₐ, R₇, R₈, R₉, R₁₀, sont tels que définis ci-dessus,
et plus particulièrement les composés de formule (Ib-1) suivants :
- composé G : R₇ = Cl, R₈ = R₉ = R₁₀ = H,
- composé H : R₇ = R₈ = R₉ = R₁₀ = H,
- composé I : R₈ = Cl, R₇ = R₉ = R₁₀ = H,
- composé J : R₉ = Cl, R₇ = R₈ = R₁₀ = H,
- composé K : R₁₀ = Cl, R₇ = R₈ = R₉ = H,
- composé L : R₉ = Br, R₇ = R₈ = R₁₀ = H.

L'invention a également pour objet les composés de formule (I) susmentionnée dans laquelle R₅ représente un ester de formule COOR' dans laquelle R' représente un groupe alkyle linéaire ou substitué de 1 à 10 atomes de carbone, notamment un groupe éthyle.

L'invention a plus particulièrement pour objet les composés de formule (Ia) susmentionnée dans laquelle :
- R₁ et R₂ représentent un atome d'hydrogène, ou forment en association avec C₁ et C₂ un cycle aromatique à 6 atomes de carbone,
- R₅ représente un ester de formule COOR' dans laquelle R' représente un groupe alkyle linéaire ou substitué de 1 à 10 atomes de carbone, notamment un groupe éthyle,
- Y représente un groupe -OH, -SH, -NH₂, ou -NHCOCH₃,
- R₇, R₈, R₉ et R₁₀ représentent un atome d'hydrogène, ou l'un au moins de R₇, R₈, R₉ ou R₁₀, représente un atome d'halogène, notamment un atome de chlore, de brome ou de fluor,
- X représente un atome d'halogène sous forme anionique, notamment un atome de brome Br, ou de chlore Cl⁻, ou un groupe d'atomes sous forme anionique.

L'invention a plus particulièrement pour objet encore les composés de formule (Ia) susmentionnée dans laquelle R₅ représente un ester de formule COOR' dans laquelle R' représente un groupe alkyle linéaire ou substitué de 1 à 10 atomes de carbone, notamment un groupe éthyle, et Y représente un groupe -OH.

A ce titre, l'invention concerne plus particulièrement les composés de formule (la) susmentionnée, et répondant aux formules suivantes :

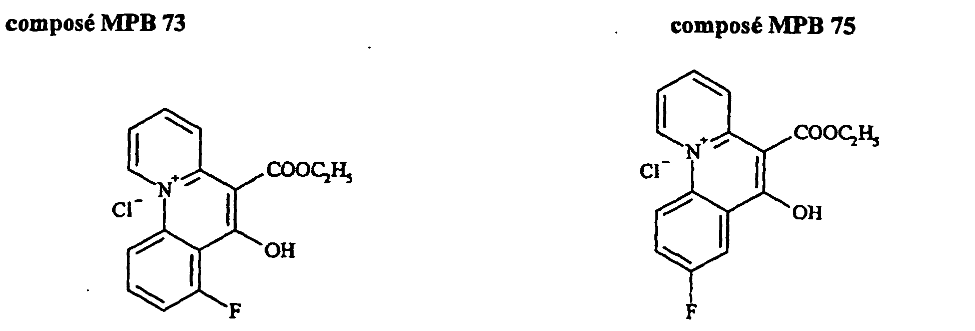

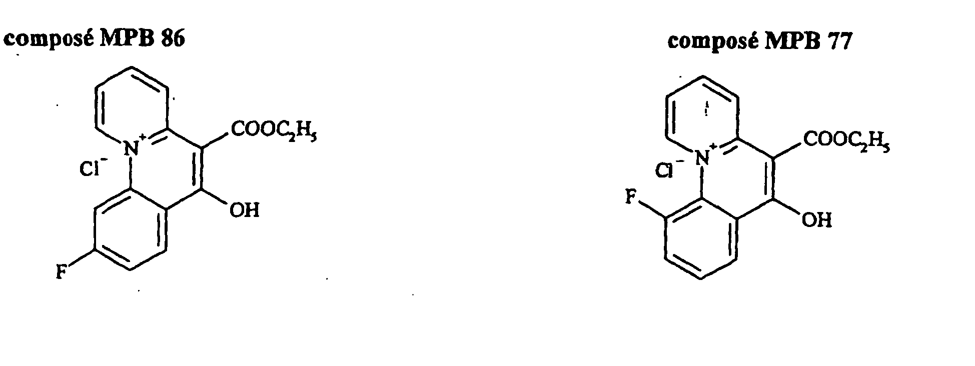

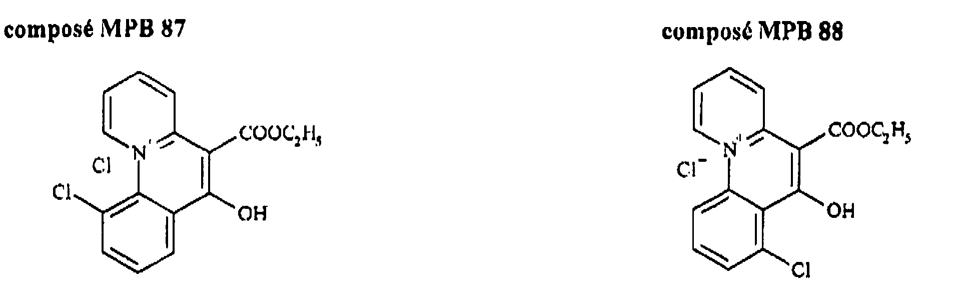

L'invention a également pour objet les composés de formule (I) susmentionnée dans laquelle Y représente SH.

L'invention a plus particulièrement pour objet les composés de formule (Ia) susmentionnée dans laquelle :
- R₁ et R₂ représentent un atome d'hydrogène, ou forment en association avec C₁ et C₂ un cycle aromatique à 6 atomes de carbone,
- R₅ représente un atome d'hydrogène, ou un groupe alkyle linéaire ou substitué de 1 à 10 atomes de carbone, notamment un groupe butyle,
- Y représente un groupe -SH,
- R₇, R₈, R₉ et R₁₀ représentent un atome d'hydrogène, ou l'un au moins de R₇, R₈, R₉ ou R₁₀, représente un atome d'halogène, notamment un atome de chlore, de brome ou de fluor,
- X représente un atome d'halogène sous forme anionique, notamment un atome de brome Br⁻, ou de chlore Cl⁻, ou un groupe d'atomes sous forme anionique,

A ce titre, l'invention concerne plus particulièrement les composés de formule (Ta) susmentionnée, et répondant aux Permutes suivantes :

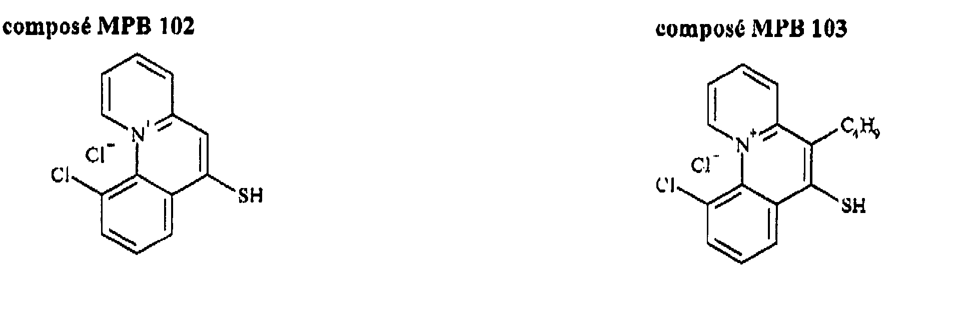

L'invention a également pour objet toute composition pharmaceutique comprenant, à titre de principe(s) actif(s), au moins un des composés de formule générale (I) décrite ci-dessus, et plus particulièrement au moins un des composés de formule (Ia) susmentionnée dans laquelle R₅ représente un ester de formule COOR' dans laquelle R' représente un groupe alkyle linéaire ou substitué de 1 à 10 atomes de carbone, notamment un groupe éthyle, et/ou Y représente SH, en association avec un véhicule physiologiquement acceptable.

A ce titre, l'invention a plus particulièrement pour objet les compositions pharmaceutiques susmentionnées comprenant au moins le composé MPB 73, MPB 75, MPB 86, MPB 77, MPB 87, MPB 88, MPB 102, ou MPB 103, en association avec un véhicule physiologiquement acceptable.

Des compositions pharmaceutiques préférées utilisées dans le cadre de la présente invention sont celles comprenant le composé 19 (encore désigné MPB-07), ou le composé MPB-91, le cas échéant en association avec un (ou plusieurs) autre(s) composé(s) de l'invention décrit(s) ci-dessus.

Avantageusement les compositions pharmaceutiques selon l'invention se présentent sous une forme administrable par voie orale, notamment sous forme de comprimés, ou de gélules, ou sous une forme administrable par voie parentérale, notamment sous forme de préparations injectables par voie intraveineuse, intramusculaire, ou sous-cutanée, ou encore par voie aérienne, notamment par voie pulmonaire sous forme d'aérosols.

Avantageusement encore, les compositions pharmaceutiques selon l'invention, sont caractérisées en ce que les quantités de principe(s) actif(s) sont telles que la posologie journalière en principe(s) actifs) est d'environ 0,1 mg/kg à 5 mg/kg, notamment d'environ 3 mg/kg, en une ou plusieurs prises.

S'agissant de la synthèse des composés de formule (I) de l'invention, celle-ci est amplement détaillée dans la demande internationale WO 98/05642, ainsi que dans l'article de Becq et al., 1999, et de Dérand et al., 2001.

L'invention sera davantage illustrée à l'aide de la description détaillée qui suit de la synthèse de nouveaux composés de l'invention, et de l'étude des composés de l'invention dans la cadre de la vasodilatation.

### I) Préparation des composés MBP 73, MPB 75, MPB 86, MPB 77, MPB 87, et MPB 88

Dans un tricol muni d'une arrivée d'azote et d'une ampoule à brome, une solution de BuLi 0.025 mole est ajoutée goutte à goutte à la seringue à une solution de 4.03 g (0.025 mole) de 1,1,1,3,3,3-hexaméthyldisilazane dans 10 mL de THF (à -5°C). Le mélange est agité à -5°C pendant 30 min. Une solution de 3.30 g (0.02 mole) de 2-pyridylacétate d'éthyle dans 25 mL de THF est ajoutée goutte à goutte sur 2 h. Puis une solution de 0.018 mole de chlorure de benzoyle difluoré (MBP 73, MPB 75, MPB 86, MPB 77), ou dichloré (MPB 87, et MPB 88) dans 10 mL de THF est ajoutée sur 1 h. Le mélange est maintenu sous agitation à -5°C pendant 1 h et 2 h à température du laboratoire. Après hydrolyse par 20 mL d'eau, le solvant est éliminé sous pression réduite.
Les composés cyclisés lors de l'évaporation du solvant de réaction, sont purifiés par chromatographie colonne sur gel de silice, élution par l'acétate d'éthyle.
0.0003 mole des composés précédemmentobtenus sont mis en solution dans un mélange de chlorure de méthylène anhydre (5 mL) et d'éther anhydre (50 mL), l'alcool chlorhydrique est ajouté jusqu'à fin de précipitation. Le précipité est filtré sur Büchner puis rincé à l'éther anhydre.

### Chlorure de 5-carboxyéthyl-6-hydroxy-10-fluorobenzo[c]quinolizinium MPB 77

### Produit pulvérulent crème

Rendement : 50%
**Point de fusion : 134°C**
Analyse élémentaire calculée pour C₁₆H₁₃NO₃ClF, 0.5 H₂O : C, 58.10 ; H, 4.26 ; N, 4.23. Trouvé C, 58.62 ; H, 3.88 ; N, 4.16.
RMN ¹H (DMSOd₆) δ ppm, signal, n protons, attribution : 8.9, pic mal résolu, 1H, H₁ ; 8.2 à 6.9, multiplet, 6H, protons aromatiques ; 6.3, 1H, singulet, OH ; 4.3, quadruplet (J=7 Hz), 2H, CH₂, 1.3, triplet (J=7 Hz), 3H, CH₃.
**IR** (KBr) ν cm⁻¹, attribution: 3420 OH, 3171 3080 C=C-H, 2973 C-C-H, 1718 C=O, 1628, 1560, 1520, 1506, 1458, 1312, 1284, 1267, 1193, 1175, 1026, 824, 774.

### Chlorure de 5-carboxyéthyl-6-hydroxy-9-fluorobenzo[c]quinolizinium MPB 86

Produit pulvérulent jaune
Rendement : **63%**
**Point de fusion** : 154°C
Analyse élémetaire calculée pour C₁₆H₁₃NO₃ClF : C, 59.73 ; H, 4.07 ; N, 4.35. Trouvé C, 59.58 ; H, 4.10 ; N, 4.28.
RMN ¹H (DMSOd₆) δ ppm, signal, n protons, attribution : 9.2, doublet (J=8 Hz), 1H, H₁; 8.7 à 7.1, multiplet, 6H, protons aromatiques ; 5.7, singulet échangeable, 1H, OH ; 4.3, quadruplet (J=7 Hz), 2H, CH₂; 1.3, triplet (J=7 Hz), 3H, CH₃.
IR (KBr) v cm⁻¹, attribution : 3454 OH, 3104 3047 3016 C=C-H, 2960 C-C-H, 1727 C=O, 1609, 1535, 1471, 1435, 1323, 1308, 1259, 1215, 1108, 1017, 831, 764.

### Chlorure de 5-carboxyéthyl-6-hydroxy-8-fluorobenzo[c]quinolizinium MPB 75

**Produit pulvérulent jaune**
Rendement : **28%**
**Point de fusion :** 168°C
**Analyse élémentaire** calculée pour C₁₆H₁₃NO₃ClF : C, 59.73 ; H, 4.07 ; N, 4.35. Trouvé C, 59.54 ; H, 4.07 ; N, 4.24.
RMN ¹H (DMSOd₆) δ ppm, signal, n protons, attribution : 9.4, doublet (J=9 Hz), 1H, H₁ ; 8.9 à 8.7, multiplet, 1H, proton aromatique ; 8.2 à 7.0, multiplet, 5H, protons aromatiques ; 6.0, singulet échangeable, 1H, OH ; 4.3, quadruplet (J=8 Hz), 2H, CH₂; 1.3, triplet (J=8 Hz), 3H, CH₃.
**IR** (KBr) ν cm⁻¹, attribution : 3500 OH, 3157 3049 C=C-H, 2960 C-C-H, 1670, 1626, 1590, 1532, 1503, 1457, 1323, 1259, 1209, 1006, 885, 792.

### Chlorure de 5-carboxyéthyl-6-hydroxy-7-fluorobenzo[c]quinolizinium MPB 73

**Produit pulvérulent crème**
Rendement : **27%**
Point de fusion : 173°C
**Analyse élémentaire** calculée pour C₁₆H₁₃NO₃ClF, 0.5 H₂O : C, 58.10 ; H, 4.26 ; N, 4.23. Trouvé C, 57.99 ; H, 4.12 ; N, 4.97.
RMN ¹H (DMSOd₆) δ ppm, signal, n protons, attribution : 9.0, doublet (J=8 Hz), 1H, H₁; 8.4 à 6.8, multiplet, 7H, protons aromatiques + OH; 4.2, quadruplet (J=7 Hz), 2H, CH₂; 1.3, triplet (J=7 Hz), 3H, CH₃.
**IR** (KBr) ν cm⁻¹, attribution : 3420 OH, 3160 3101 3046 C=C-H, 2984 C-C-H, 1720 C=O, 1646, 1617, 1601, 1501, 1446, 1409, 1347, 1323, 1274, 1227, 1134, 1011, 814, 779.

### 5-n-Butyl-10-chloro-6-hydroxybenzo[c]quinolizinium chloride (MPB-91)

Le 2-pentylpyridine a étéobtenu dans un premier temps par condensation du 2-picolyllithium avec le 1-bromobutane à -40°C, et purifié par chromatographie sur colonne de gel de silice à l'aide de dichloromethane en tant qu'éluent. Puis, le 2-pentylpyridine 4.48 g (0.03 mol) en solution dans du THF (30 mL) a été traité à 0°C avec du diisopropylamide de lithium (0.033 mol), refroidi à -40°C, et le 2,3-dichlorobenzoate de méthyle 3.08 g (0.015 mol) dans du THF (15 mL) a étéajouté. Le mélange a étéagité 1 h à -40°C et hydrolysé à 20°C avec 10 mL d'eau. La phase organique a étéséchéesur Na₂SO₄, concentré sous vide et purifiée par chromatographie sur colonne avec de l'éther de pétrole et de l'acétated'éthyle en tant qu'éluent. Le produit pur a été chauffé à 200°C pendant 1 h. Le résidu a été chromatographié sur florisil avec du dichloromethane afin d'obtenir la cétone cyclique. Cette dernière a étésolubilisée dans l'éther anhydre et une solution de HCl/éthanol a étéadditionnée goutte à goutte. Produit pulvérulent crème ; point de fusion = 160°C, rendement: 15 %. Anal. C₁₇H₁₇N₁O₁Cl₂: C, 63.37; H, 5.32; N, 4.35; Trouvé: C, 63.36; H, 5.27; N, 4.32. IR (KBr): 3439, 3121., 2951, 2923, 2895, 2863, 2496, 2344, 1629, 1589, 1502, 1488, 1457, 1400. ¹H NMR (Me₂SO-*d*₆): σ 9.25 (δ, J = 7 Hz, 1 H, H1), 8.5 (d, J = 7 Hz, 1 H), 8.1-6.9 (m, 5H + OH), 2.9 (t, J = 4 Hz, 2H, CH₂), 1.5-1.3 (m, 4H, 2 CH₂), 0.9 (t, 3 H, CH₃). Spectre de masse (ESI): 286 (M-HCl).

### II) Effets pharmacologiques des composés de l'invention

Les effets pharmacologiques des composés de la famille des benzo[c]quinolizinium, activateurs de CFTR: le chlorure de 6-hydroxy-10-chlorobenzo[c]quinolizinium (nom de référence : MPB-07) et de 5-butyl-6-hydroxy-10-chlorobenzo[c]quinolizinium (nom de référence : MPB-91), ont été étudiés sur des anneaux d'aorte isolée de rat et d'artères mammaires humaines.

Les résultats montrent les faits suivants
(1)MPB-91 (EC₅₀ = 21 µM) et MPB-07 (EC₅₀ = 134 µM) ont un effet vasorelaxant dose-dépendant, réversible et lent, sur les deux préparations.
(2) Ces composés activent un transport anionique dans les cellules musculaires lisses. Le profil d'activation de ce transport (MPB-91, MPB-07) est similaire à celui rencontré dans les cellules épithéliales exprimant la protéine CFTR.

### Effets vaso-dilatateur des composés de la famille benzo[c]quinolizinium.

### MATERIEL ET METHODES

### A. Matériel biologique

### Prélèvement et dissection de l'aorte de Rat

Les expérimentations sont réalisées sur l'aorte thoracique de rats mâles de souche Wistar de 200 à 250 g âgés de 7 à 8 semaines. Le rat est assommé puis placé en décubitus dorsal. Après thoracotomie, le segment d'aorte compris entre le diaphragme et la crosse aortique est excisé. Le morceau d'aorte est immédiatement placé dans une solution physiologique tamponnée de type Krebs modifié. L'aorte est sectionnée en anneaux de 5 mm.

### Abrasion de l'endothélium

Pour certaines préparations, l'endothélium est enlevé mécaniquement à l'aide d'une aiguille entourée de fil que l'on fait tourner dans l'anneau d'aorte. Ce procédé ne lèse pas les cellules musculaires lisses.

### B. Technique des organes isolés

### Montage dans la cuve à organes isolés

Les anneaux d'aorte, après dissection, sont suspendus entre deux crochets dont l'un est fixe et l'autre mobile. Ce montage est placé dans une cuve en Plexiglas (Emka Technologies), thermostatée à 37°C, contenant 5 ml de solution physiologique oxygénée par un mélange gazeux (95% O₂ - 5% CO₂). L'enceinte thermostatée est alimentée par un bain-marie. Le crochet mobile est suspendu à un capteur relié à l'interface (transducteur mécanoélectrique). Cette interface est reliée d'une part à un amplificateur (amplification du signal) et d'autre part à un ordinateur (visualisation et analyse du signal). Le programme IOX 1.565 permet le traitement et la visualisation des signaux sur l'écran d'ordinateur. Les programmes Data Analyst et Prism 3.0 permettent l'analyse des résultats.

### Principe de fonctionnement

La force isométrique développée par les préparations artérielles est transformée en une différence de potentiel qui est amplifiée puis transcrite en un signal visualisé sur un écran d'ordinateur.

### Méthodologie

Après le montage des anneaux d'aorte dans le dispositif expérimental, ceux-ci sont étirés à une tension basale de 2.5 g. Cette tension correspond au point optimum de leur relation tension/longueur. L'attente de l'état stable dans le milieu physiologique de référence se réalise sur une période d'environ 1 heure avec un lavage toutes les 20 minutes. A la suite de cette période d'équilibration, les différentes expériences sont réalisées.

### Test de la fonction endothéliale

Afin de s'affranchir des mécanismes de relaxation/contraction dus à l'endothélium, certaines préparations sont débarrassées de celui-ci par destruction mécanique. Pour s'assurer de la destruction effective de l'endothélium, un test cholinergique (ACh 10-4 M) est réalisé au terme de la première période d'équilibration, sur des préparations précontracturées par une solution hyperpotassique (80 mM de KCl). Ce test est considéré valide si l'ajout d'ACh ne provoque pas de relaxation et que la préparation reste à l'état contractée.

Suite à ce test, les préparations subissent plusieurs lavages successifs afin de retrouver un état stable. Une fois l'état stable atteint, les différentes expériences peuvent être réalisées. Les contractures dues aux solutions hyperpotassiques sont obtenues par remplacement total de la solution physiologique de référence par la solution hyperpotassique. L'administration des substances pharmacologiques se fait de façon cumulative et directement dans les cuves à organes isolés en prenant garde que le volume additionnel ne soit pas trop élevé. L'application de chaque nouvelle concentration ne se fait qu'après stabilisation. Afin de rendre compte de l'état de réactivité de la préparation en fin de manipulation, un test hyperpotassique ou noradrénergique est effectué. Si la préparation réagit dans le sens d'une contracture, le test est validé.

### C. Les différentes solutions physiologiques et les substances pharmacologiques utilisées

### Solutions physiologiques

La composition de la solution physiologique de référence est une solution de type Krebs modifié : NaCl: 120mM ; KCl: 4,7mM ; CaCl₂ : 2,5mM ; MgCl₂ : 1,2mM ; NaH₂PO₄ : 1,2mM ; NaHCO₃ : 15mM ; Hepes : 10mM ; Glucose : 11mM ; pH = 7,4. Des solutions hyperpotassiques contenant différentes concentrations de KCl ont été préparées selon le même principe. Ces solutions sont utilisées pour provoquer une contracture de l'anneau d'aorte par dépolarisation des cellules musculaires lisses. La solution hyperpotassique majoritairement utilisée dans les expériences contient 80 mM de KCl.

### Substances pharmacologiques

- MPB-07 (chlorure de 6-hydroxy-10-chlorobenzo[c]quinolizinium)
- MPB-91 (chlorure de 5-butyl-6-hydroxy-10-chlorobenzo[c]quinolizinium)
- Artérénol (norépinephrine, SIGMA, USA)
- ACh (acétylcholine, SIGMA, USA)
- L-NAME (N░-nitro-L-arginine methyl ester, SIGMA, USA)
- MPB-70 (10-chlorobenzo[c]quinolizine-6-one)
- Vérapamil (SIGMA, USA)

### D. Les efflux d'iodure

La technique des efflux d'iodure, mise au point par Venglarick et al. (1990), permet la mesure des mouvements d'ions et est décrite en détail dans Dérand et al. (2001).

### Méthodologie

Les anneaux d'aorte de taille équivalente, sont incubés dans 1 ml de solution de charge composée de solution physiologique de type Krebs modifié ou hyperpotassique (80 mM KCl) supplémentée de 1,5 µl/ml de Na ¹²⁵I et 0,1 µl/ml de KI. Après 1 h 30 d'incubation à 37°C, 4 rinçages successifs de deux minutes sont réalisés dans 1 ml de solution physiologique appropriée (solution Krebs normale ou hyperpotassique). Une cinétique d'efflux d'iodure des anneaux d'aorte est réalisée pendant 12 min. Après chaque minute, le contenu de chaque puits est prélevé et placé dans des tubes à hémolyse, et la radioactivité est comptée par un compteur gamma (Packard, Cobra II). A la 12^{e} minute, les anneaux d'aorte sont solubilisés dans du Dodécyl Sulfate de Sodium (SDS 0,1%) + NaOH (0,1M). Cette étape permet la récupération de la radioactivité résiduelle par destruction cellulaire.

### Présentation des résultats

La radioactivité totale des cellules est calculée par l'addition des coups par minute (cpm) de chaque fraction collectée et des cpm de la fraction solubilisée (radioactivité résiduelle). Les courbes sont tracées en exprimant les efflux en pourcentage du contenu cellulaire accumulé dans le milieu en fonction du temps. Par exemple, la valeur attribuée au temps tₙ, correspond à : 100 × (Σ cpm obtenus de t₀ à tₙ )/radioactivité totale. La vitesse instantanée de sortie est présentée selon la formule Ln(¹²⁵It₁/¹²⁵It₂)/(t₁-t₂), où t₁ et t₂ sont deux temps successifs et ¹²⁵It₁ et ¹²⁵It₂ sont les efflux mesurés aux temps t₁ et t₂. Pour évaluer l'effet d'une substance, on détermine le rapport d'activation r = vitesse de sortie après stimulation / vitesse de sortie avant stimulation. Les résultats (moyenne ± SEM) sont comparés à l'aide du test statistique de Student pour une valeur appariée.

### RESULTATS

### A. ORGANES ISOLES

### 1 / Expériences témoins

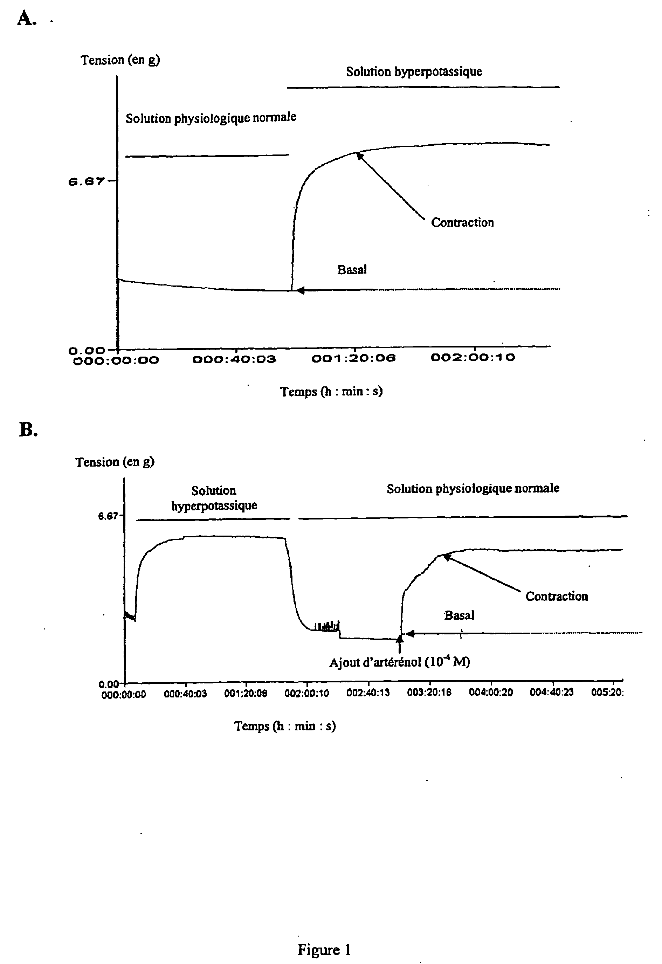

■ **Contracture hyperpotassique et contracture par l'artérénol *(figure 1A B)***
   Après un état de stabilité des anneaux d'aorte en solution normale de type Krebs, la totalité de la solution est remplacée par une solution hyperpotassique contenant 80 mM de KCl. Cette solution a pour effet de contracter les anneaux d'aorte par dépolarisation des cellules musculaires lisses. La figure 1A montre que la contracture obtenue en milieu hyperpotassique est stable dans le temps. Elle est prise comme contrôle (100% de la contracture) dans les différentes expériences réalisées. La contracture par l'artérénol (10⁻⁴ M) se fait par ajout directement dans la cuve contenant la solution normale de type Krebs. Le mécanisme qui conduit à la contracture de l'aorte est différent. L'artérénol se fixe suc un récepteur adrénergique de type alpha 1 intégré à la membrane des cellules musculaires lisses. C'est la voie classique via le DAG et l'IP₃ qui permet la contracture.
■ **Validation de la technique de désendothélisation**
   Afin de vérifier la présence ou non de l'endothélium, le test cholinergique est utilisé. Après contracture de l'anneau d'aorte en milieu hyperpotassique (80 mM de KCl), de l'ACh (10⁻⁴ M) est ajouté dans la cuve. Les résultats montrent que les anneaux d'aorte *sans endothélium* restent contracté alors que les anneaux d'aorte *avec endothélium* se relaxent (environ 40% de relaxation). Dans cette étude ce test a été validé.
■ **Effets de différents solvants, solutions et substances**
   Avant de commencer .les expérimentations, différents tests témoins de diverses substances ont été pratiqués afin d'anticiper leurs effets éventuels sur les préparations. L'ajout d'eau et de DMSO (diméthylsulfoxyde), solvants principaux de la majorité des substances injectées aux préparations, n'ont aucun effet sur les anneaux d'aorte, que ceux-ci soient en solution Krebs normale ou en solution hyperpotassique.
■ **Contrôle du pH**
   Les solutions Krebs normale ou hyperpotassique ont un pH de 7,4. Sur une expérience de 7 heures, ce pH ne présente aucune dérive, dans les deux conditions. Après ajout de MPB-07 ou autres substances pharmacologiques utilisées, le pH ne présente aucune dérive dans le temps.
■ **Dérive de la contracture des anneaux d'aorte dans le temps**
   Les anneaux d'aorte précontracturés soit par une solution hyperpotassique soit par de l'artérénol, présentent une contracture stable dans le temps, une heure environ après l'état stable de contracture acquis.

### 2/ Effet du MPB-07 sur des anneaux d'aorte précontracturés en solution hyperpotassique

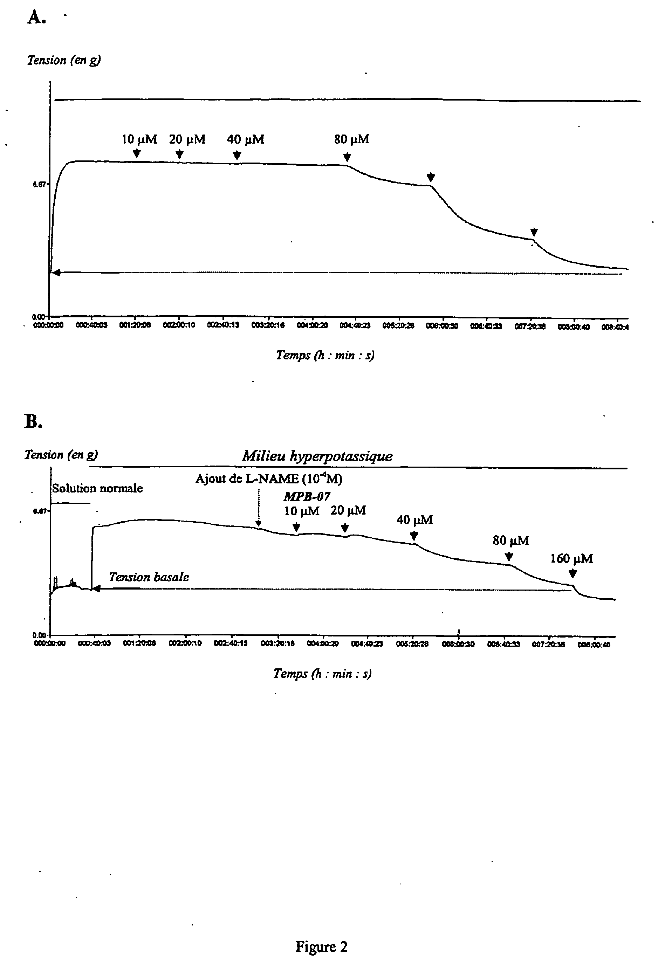

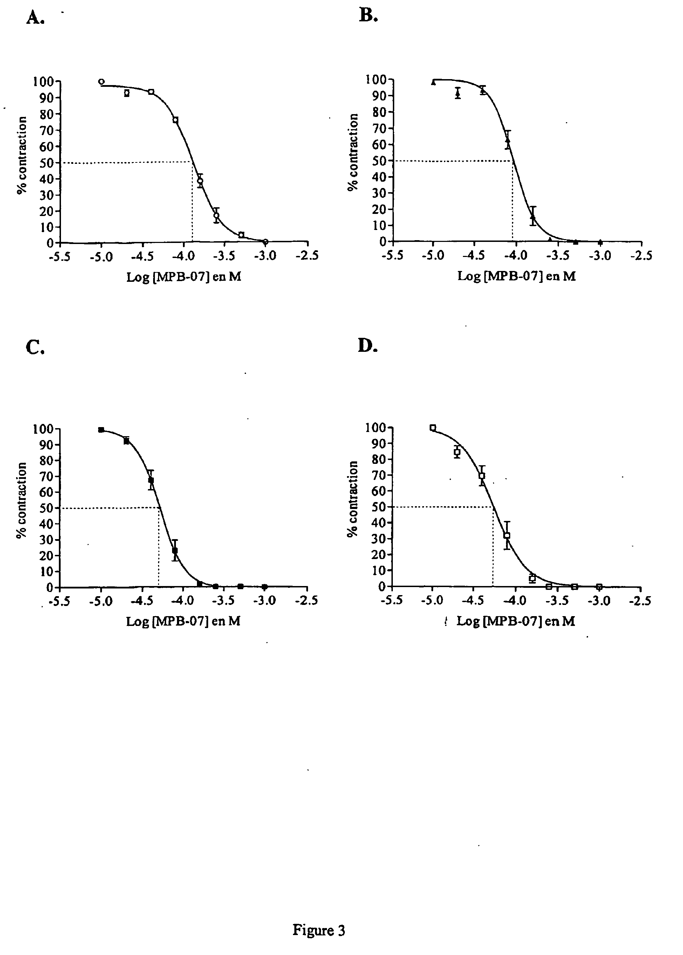

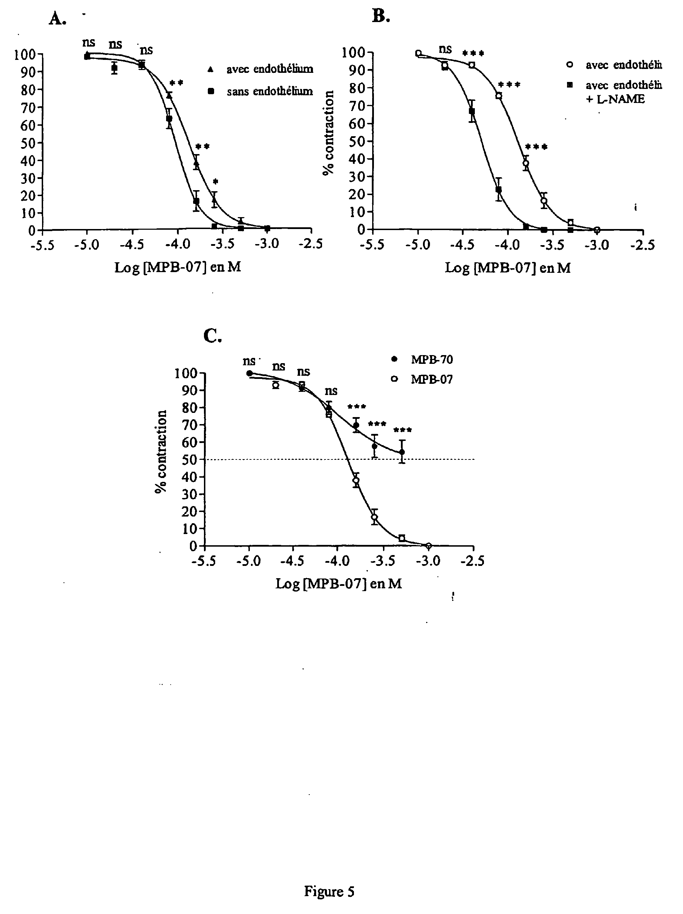

■ **Anneaux d'aorte avec endothélium** *(figure 2 A figure 3A)*
   Le MPB-07 provoque une relaxation dose-dépendante des anneaux d'aorte avec endothélium comme le montre la figure 2A. Les résultats présentés figure 3A sont en pourcentage de la contracture maximale obtenue en solution hyperpotassique. La figure 3A montre que sur des anneaux d'aorte avec endothélium, le MPB-07 présente une EC₅₀ = 132,4 ± 0,02 µM (n =14). La relaxation est effective à partir de 80 µM (24 ± 1,6 % de relaxation, n =14 ) et atteint 96 ± 1,75% (n =14 ) pour 500 µM.
■ **Anneaux d'aorte sans endothélium** *(figure 3B)*
   Le MPB-07 provoque une relaxation dose-dépendante des anneaux d'aorte sans endothélium. Cette molécule a donc une action endothélium indépendante. Les résultats sont présentés en pourcentage de la contracture maximale obtenue en solution hyperpotassique. Sur les anneaux d'aorte sans endothélium, la figure 3B montre que le MPB-07 présente un EC₅₀ = 92,90 ± 0,02 µM ( n =7 ). La relaxation est probante à partir de 80 µM ( 37 ± 5,6% de relaxation, n =7 ) et atteint 99 ± 1 % ( n =7 ) à 250 µM. En comparaison avec le résultat précédent, le MPB-07 semble plus efficace sur les anneaux d'aorte *sans endothélium* que sur les anneaux d'aorte *avec endothélium,* les deux EC₅₀ sont significativement différents (test de Student, p<0,01). L'absence de l'endothélium joue donc un rôle sur l'effet relaxant provoqué par le MPB-07.
■ **Anneaux d'aorte avec endothélium + L-NAME** *(figure 2B ; figure 3C)*
   La L-NAME a été utilisée afin d'inhiber les mécanismes de régulation par l'endothélium sur les cellules musculaires lisses et dans le but de mimer une absence de l'endothélium. Cette molécule est un inhibiteur de la NO (nitric oxyde) synthase présente dans les cellules endothéliales. La L-NAME (10⁻⁴ M) est ajoutée dans la solution hyperpotassique 30 minutes avant la gamme de MPB-07. Le MPB-07 provoque une relaxation dose-dépendante des anneaux d'aorte avec endothélium traités par la L-NAME comme le montre la figure 2B. Les résultats (figure 3C) sont présentés en pourcentage de la contracture maximale obtenue en solution hyperpotassique après l'ajout de la L-NAME. Le MPB-07 présente un EC₅₀ = 51,80 ± 0,008 µM ( n =8 ). La relaxation se prononce à partir de 40 µM ( 33 ± 6,15% de relaxation, n =8 ) et atteint 98 ± 1,3% pour 160 µM. En comparaison avec les résultats précédents, la présence de L-NAME potentialise l'effet vasorelaxant du MPB-07.
■ **Anneaux d'aorte sans endothélium en présence de L-NAME** *(figure 3D)*
   L'effet du MPB-07 a été testé sur des anneaux d'aorte sans endothélium et en présence de L-NAME pour bloquer la voie de régulation par le NO. Dans cette situation, le MPB-07 provoque une relaxation dose-dépendante des anneaux d'aorte avec un EC₅₀ = 55,1 ± 0,2 µM (n =7). Il est à noter que les courbes « avec endothélium + L-NAME » et «sans endothélium + L-NAME » sont comparables. Les figures 5A et 5B présentent les différences statistiques entre la courbe «avec endothélium » et les courbes « sans endothélium » et « avec endothélium + L-NAME ».

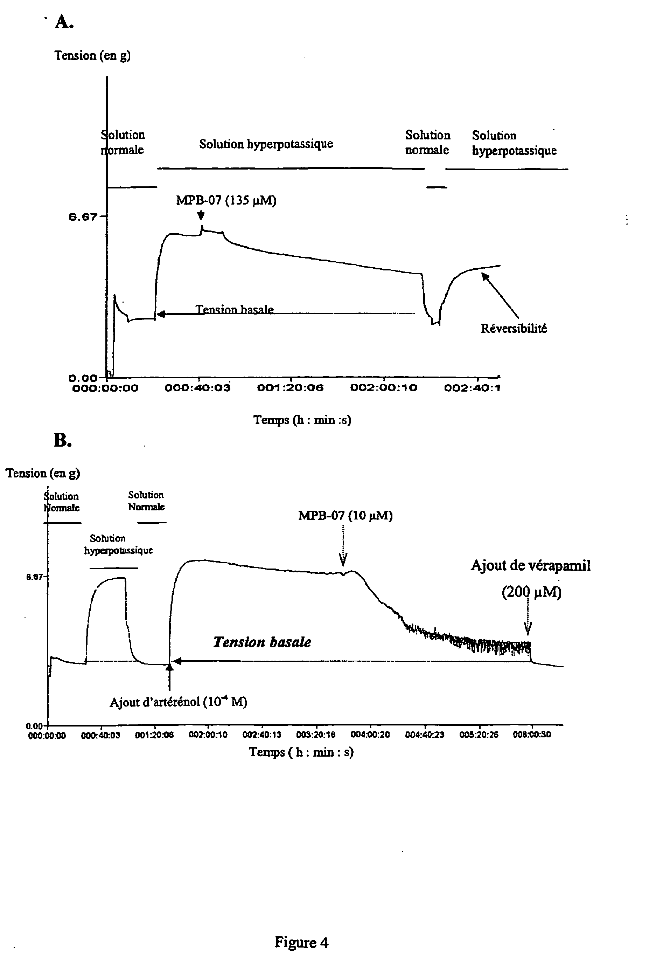

### 3/ Réversibilité de l'effet MPB-07 (figure 4A)

Nous venons de montrer que le MPB-07 induisait une vasorelaxation des anneaux d'aorte précontracturés en solution hyperpotassique. Cet effet est réversible comme le montre la figure 4A. Dans cette expérience l'ajout de MPB-07 en dose unique (135µM) provoque une relaxation des anneaux d'aorte avec endothélium précontracturés en milieu hyperpotassique. Après un état stable de relaxation, les anneaux sont lavés en milieu physiologique normal afin de revenir à l'état basal de contracture, puis contracté de nouveau en milieu hyperpotassique. Les résultats (n = 4) montrent, une réactivité positive des anneaux d'aorte, après une dose unique de MPB-07, ceux-ci retrouvant leur contracture hyperpotassique initiale. Ces résultats montrent la réversibilité de l'effet relaxant du MPB-07.

### 4/ Effet du MPB-07 sur des anneaux d'aorte précontracturés par de l'artérénol

La figure 4B montre que l'ajout de MPB-07 (10 µM) provoque une relaxation des anneaux d'aorte avec endothélium précontracturés par de l'artérénol (10⁻⁴M). Les résultats montrent 90 ± 9% de relaxation au bout de 2h30. Des oscillations rapides de contracture sont observables. Ces variations transitoires présentent une amplitude qui augmente en fonction du temps. L'ajout de vérapamil (inhibiteur canaux Ca _{L}, 200 µM) stoppe ces variations.

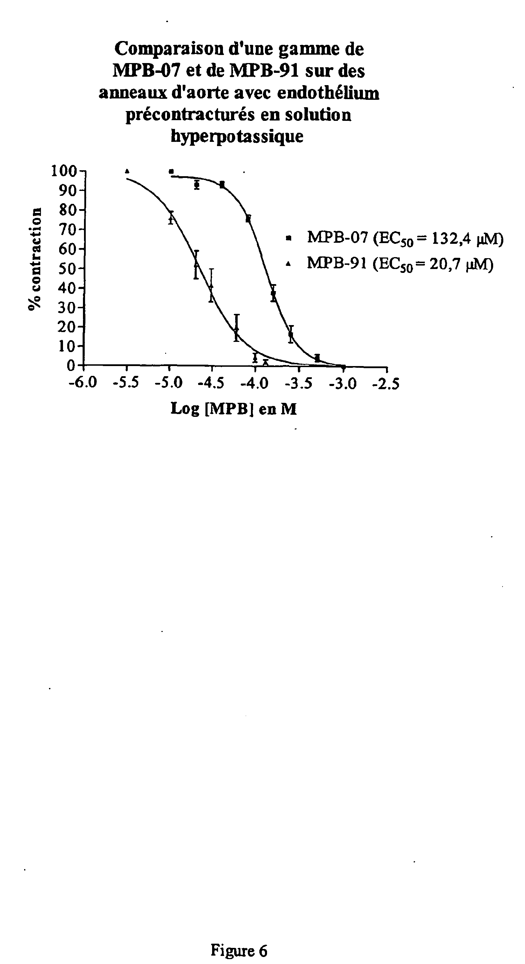

### 5/ Comparaison de l'effet du MPB-07 avec l'effet du MPB-91 et MPB-70 (figure 5C et 6)

Le MPB-07 et MPB-91 sont des activateurs de CFTR. Il nous est paru intéressant de comparer leur effets avec le MPB-70 un analogue inactif sur CFTR des cellules épithéliales, et dont la formule est la suivante

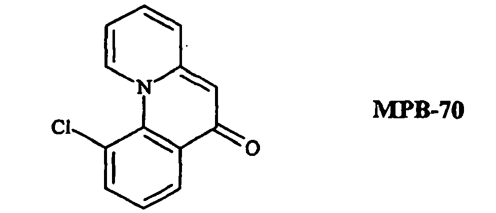

De 10 µM à 80 µM l'effet des 2 molécules est semblable, les résultats ne présentent pas de différence statistiquement significative. A 160 µM, le MPB-70 relaxe les anneaux d'aorte (n = 7) de 27 % contre 64 % par le MPB-07 (n = 14) : cette différence est significative (*** ; p<0,001). A 250 µM, le MPB-70 provoque 42 % de relaxation des anneaux d'aorte alors que le MPB-07 à cette même concentration provoque 88 % de relaxation (différence statistiquement significative, p<0,001). A 500 µM, le MPB-07 relaxe à 100% les anneaux d'aorte alors que le pourcentage de relaxation par le MPB-70 ne varie peu (46 %) et reste le même pour des concentrations supérieures. La comparaison des courbes doses/réponses présentée figure 6 montre que le MPB-91 (EC₅₀ = 21 µM) est plus efficace que le MPB-07 (EC₅₀ = 133 µM).

### 6/ Conclusion

Cette étude montre que :
➢ Le MPB-07 et le MPB-91 ont un effet vasorelaxant dose-dépendant, réversible et lent, sur les anneaux d'aorte précontracturés.
➢ L'inhibition de la voie de la NO-synthase par la L-NAME renforce cet effet pharmacologique.
➢ L'ordre d'efficacité des composés est MPB-91>MPB-07>>MPB-70 sur ces préparations suit celui observé sur le CFTR épithélial humain.
➢

### B. LES EFFLUX D'IODURE

La technique des efflux d'iodure a été utilisée pour caractériser les transports anioniques et leur mode de stimulation par le MPB-07. Cette technique permet de tracer un transport ionique dans une cellule chargée au préalable avec le radiotraceur correspondant. Nous avons adapté cette technique utilisée en routine au laboratoire pour l'étude sur des anneaux d'aorte.

Nous avons d'abord étudié les cinétiques de sortie de ¹²⁵I des cellules musculaires lisses des anneaux d'aorte (dépourvus d'endothélium) à l'état basal, c'est-à-dire en absence de stimulation. Ces expériences ont été réalisées dans deux conditions : en milieu physiologique normal et en milieu hyperpotassique. Cette étude permet de comparer les résultats obtenus après stimulation des anneaux d'aorte. Les résultats montrent que la sortie d'iodure s'effectue régulièrement en fonction du temps. L'efflux basal correspond à la diffusion passive de la charge radioactive en dehors des cellules. Ces expériences montrent que l'absence de vitesse de sortie correspond à l'absence de stimulation d'un transport anionique.

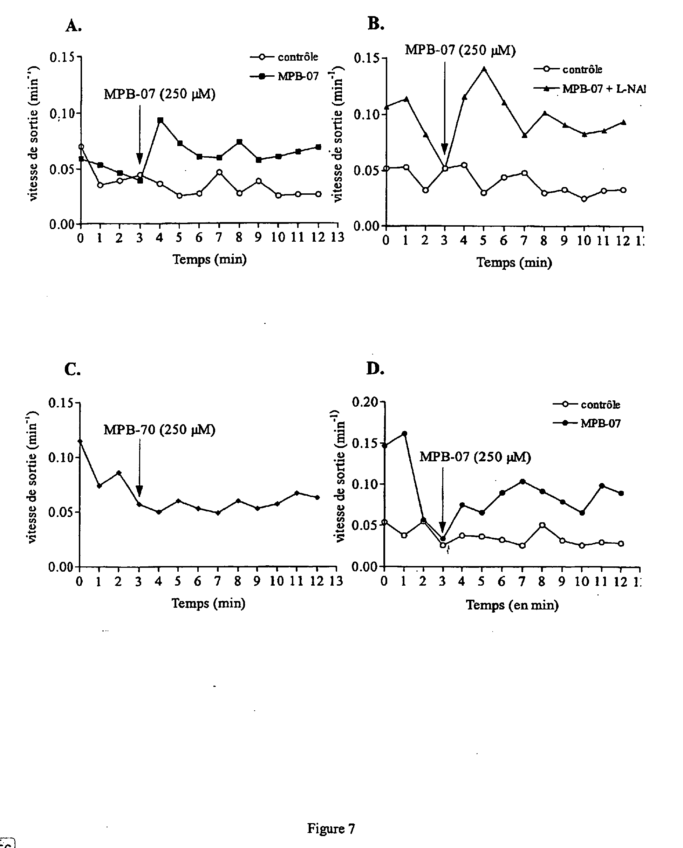

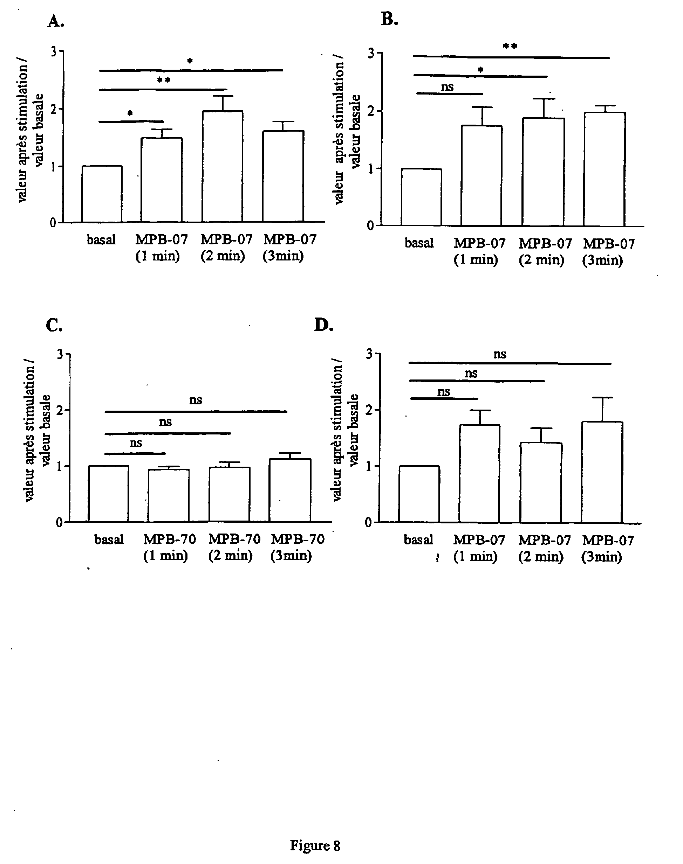

### 1/ Effet du MPB-07 sur les efflux d'iodure, sur des anneaux d'aorte sans endothélium en milieu hyperpotassique (80 mM KCl) (figure 7A et 8A)

Dans une même situation expérimentale que celle utilisée en organes isolés l'ajout de MPB-07 provoque une augmentation de l'efflux d'iodure comme le montre la figure 7A. La vitesse de sortie augmente de 0,053 ± 0,005 à l'état basal à 0,076 ± 0,008 (augmentation de 0,023) une minute après l'addition de MPB-07, puis 0,099 ± 0,013 (soit une augmentation de 0,046) 2 minutes après l'addition de la molécule (résultats de n = 8 expériences). Ces résultats montrent que le MPB-07 active un transport anionique dans ces conditions. La figure 8A montre les différences statistiques entre la vitesse de sortie avant et après stimulation par le MPB-07.

### 2/ Effet du MPB-07 sur les efflux d'iodure, sur des anneaux d'aorte avec endothélium traités à la L-NAME, en milieu hyperpotassique (figure 7B et 8B)

L'ajout de MPB-07 provoque une augmentation de l'efflux (figure 7B). En présence de

L-NAME (10⁻⁴ M) cette augmentation est plus élevée que dans l'expérience précédente. En effet, la vitesse de sortie passe de 0,059 à l'état basal à 0,103 ± 0,017 (augmentation de 0,044) une minute après l'addition de MPB-07, puis à 0,110 ± 0,015 (augmentation de 0,051) 2 minutes après l'ajout de MPB-07, pour atteindre 0,119 ± 0,011 (augmentation de 0,060) 3 minutes après l'addition de la molécule (résultats de n = 4 expériences). L'augmentation de vitesse de sortie est statistiquement significative par rapport à la valeur basale (figure 8B). Ces résultats montrent que le transport anionique activé par le MPB-07 (250 µM) est potentialisé par la présence de L-NAME. L'inhibition de la NO synthase potentialise l'activation d'un transport anionique par le MPB-07. Ce résultat conforte les résultats obtenus en organes isolés.

### 3/ Effet du MPB-70 sur les efflux d'iodure, sur des anneaux d'aorte sans endothélium, en milieu hyperpotassique (figure 7C et 8C)

Le MPB-70 est une molécule de la même famille que le MPB-07, mais inactive sur le canal CFTR. Comme en organes isolés, le MPB-70 a été testé en efflux de radiotraceurs. Sur les anneaux d'aorte, l'addition de MPB-70 ne provoque pas d'augmentation de la vitesse de sortie. La vitesse de sortie passe de 0,056 ± 0,002 à 0,053 ± 0,003 une minute après l'addition de MPB-70. Cette vitesse n'atteint que 0,055 ± 0,005, 2 minutes après l'ajout de la molécule.

### 4/ Effet du MPB-07 sur les efflux d'iodure, sur des anneaux d'aorte sans endothélium en milieu physiologique normal (figure 7D et 8D)

La stimulation par le MPB-07 entraîne une faible activation de l'efflux d'iodure. Ceci se traduit par une augmentation de la vitesse de sortie qui passe de 0,044 ± 0,009 à 0,076 ± 0,016 sur n = 3 expériences. Statistiquement cette augmentation est non significative (ns) comme le montre la figure 8D.

### 5/ Conclusion

Le MPB-07 active un transport anionique dans les cellules musculaires lisses, potentialisé par la L-NAME. Le profil d'activation de ce transport (MPB-07>>>MPB-70) est similaire à celui rencontré dans les cellules épithéliales exprimant le CFTR humain. Des effets similaires ont été observé avec le composé MPB-91.

### C. Démonstration de l'expression de la protéine CFTR dans la cellule musculaire lisse de l'aorte de rat et de souris.

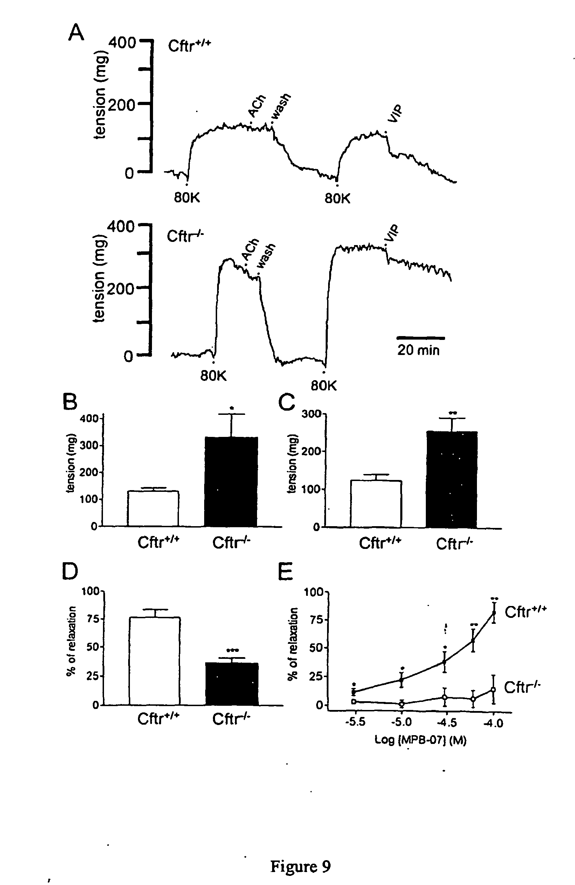

CFTR est détectée au niveau de son ARN messager et au niveau protéique dans les muscles lisses. Comme témoin négatif les inventeurs ont utilisé le muscle squelettique de rat au niveau duquel ni le messager ni la protéine ne sont présents. CFTR est détectable dans le muscle lisse par immunoprécipitation et phosphorylation *in vitro* par la protéine kinase A. La pharmacologie de CFTR a été étudiée par efflux d'iodure dans la cellule musculaire fraîchement dissociée à partir d'aorte de rat. Il est démontré par les inventeurs que des agonistes de la voie de l'AMPc comme la forskoline, l'IBMX, le cpt-AMPc et le neuropeptide VIP activent un efflux iodure dont la signature pharmacologique est celle du CFTR. Son profil d'inhibition est similaire à celui du CFTR épithélial (sensibilité au glibenclamide et au DPC mais pas au Calixarene). Dans des expériences sur tissus de rat et de souris Cftr^{+/+}, l'activation physiologique de CFTR par le VIP et pharmacologique par les composés MPB-07 et MPB-91 conduit à une relaxation de l'aorte précontractée. Chez des souris invalidées pour le gène CFTR (= souris Cftr^{-/-}, souris B6; 129-CFTR ^{tm1Unc.} en provenance du centre d'élevage CNRS CDTA d'Orléans), cet effet est très largement réduit. De plus, une contraction plus importante est observée chez les animaux KO en réponse aux agents constricteurs. La figure 9 présente ces résultats.

Ces résultats démontrent (1) que CFTR est exprimé et fonctionnel dans le muscle lisse, (2) que son profil pharmacologique tant pour l'activation que pour l'inhibition est très similaire à celui du CFTR épithélial, (3) que son absence dans la souris KO conduit à des troubles de tension vasculaire très proches de ceux rencontrés dans l'hypertension, (4) que les benzo[c]quinoliziniums représente une nouvelle famille de composés anti-hypertenseurs, ouvrant ainsi une nouvelle voie de recherche pharmacologique et de traitement potentiel des maladies liées à l'hypertension. Ces résultats démontrent en outre qu'une molécule qui se révèlerait être activatrice de CFTR, peut agir comme un vasorelaxant et un broncho-dilatateur potentiel.

### Légende des figures

**Figure 1** :
   **A.** Tracé représentatif d'une contraction d'anneaux d'aorte par une solution hyperpotassique
   **B.** Tracé représentatif d'une contraction d'anneaux d'aorte par de l'artérénol (10⁻⁴ M)
**Figure 2 :** Tracés représentatifs d'une gamme de MPB-07 : **A.** sur des anneaux d'aorte avec endothélium précontractés en solution hyperpotassique ; **B.** sur des anneaux d'aorte avec endothélium précontractés en solution hyperpotassique, en présence de L-NAME (10⁻⁴M) en préincubation 30 min avant l'ajout de MPB-07.
**Figure 3** : Courbes représentant le pourcentage de contraction des anneaux d'aorte en fonction du logarithme décimal de la concentration en MPB-07 (en M). La gamme de MPB-07 (10, 20, 40, 80, 160, 250, 500 et 1000 µM) est réalisée sur des anneaux d'aorte précontractés en solution hyperpotassique (80 mM de KCl) :
   **A.** avec endothélium, moyenne ± SEM de n = 14 expériences réalisées sur 4 rats ;
   **B.** sans endothélium, moyenne ± SEM de n = 7 expériences réalisées sur 2 rats ;
   **C.** avec endothélium, en présence de L-NAME (10⁻⁴ M) en préincubation 30 min avant l'ajout de MPB-07, moyenne ± SEM de n = 8 expériences réalisées sur 2 rats ; **D.** sans endothélium, en présence de L-NAME (10⁻⁴ M) en préincubation 30 min avant l'ajout de MPB-07, moyenne ± SEM de n = 7 expériences réalisées sur 2 rats. Le pourcentage de contraction est calculé par rapport à la valeur de tension maximale obtenue en solution hyperpotassique. Les résultats sont normalisés par rapport à cette valeur.
**Figure 4 : A.** Tracé représentatif de la réversibilité de l'effet du MPB-07 (135 µM) sur des anneaux d'aorte avec endothélium **B.** Tracé représentatif de l'effet du MPB-07 (à 10 µM) sur des anneaux d'aorte avec endothélium précontractés par de l'artérénol (10⁻⁴M).
**Figure 5 :** Comparaison statistique des doses / réponses du MPB-07 entre des anneaux d'aorte : **A.** avec endothélium et sans endothélium **B.** avec endothélium et avec endothélium en présence de L-NAME (10⁻⁴ M) ; **C.** Comparaison des doses / réponses du MPB-07 et du MPB-70 sur des anneaux d'aorte avec endothélium. Les résultats sont présentés en pourcentage de contraction en fonction du logarithme décimal de la concentration en MPB-07. Les valeurs de contraction pour chaque concentration sont comparées statistiquement, par le test de Student.
   ns : différence non significative entre les 2 valeurs comparées
   * : différence significative entre les 2 valeurs comparées, p<0,05
   ****** : différence significative entre les 2 valeurs comparées, p<0,01
**Figure 6** : Comparaison des courbes doses / réponses du MPB-07 et MPB-91: Les résultats sont présentés en pourcentage de contraction en fonction du logarithme décimal de la concentration en MPB-07.
**Figure 7 :** Résultats représentatifs des efflux d'iodure sur des anneaux d'aorte sans endothélium : **A.** en présence de MPB-07 (250 µM) en milieu hyperpotassique ; **B.** en présence de MPB-07 (250 µM) en milieu hyperpotassique et L-NAME (10⁻⁴M) en préincubation 30 min avant l'ajout de MPB-07 ; **C.** en présence de MPB-70 (250 µM) en milieu hyperpotassique ; **D.** en présence de MPB-07 (250 µM) en milieu physiologique normal.
   Le MPB-07 ou MPB-70 est ajouté au bout de 4 min. La valeur au temps 4 min est prise comme valeur contrôle. La vitesse de sortie est représentée en fonction du temps.
**Figure 8**: Représentation du rapport de la valeur de vitesse de sortie obtenue après différents temps de stimulation et de la valeur de vitesse de sortie avant stimulation.
   A. stimulation par MPB-07 en milieu hyperpotassique, moyenne ± SEM de n = 8 expériences; **B.** stimulation par MPB-07 en milieu hyperpotassique, en présence de L-NAME (10⁻⁴ M) 30 min avant l'ajout, moyenne ± SEM de n = 4 expériences ; **C.** stimulation par MPB-70, en milieu hyperpotassique, moyenne ± SEM de n = 4 expériences ; **D.** stimulation par MPB-07 en mileu physiologique normal, moyenne ± SEM de n = 3 expériences. Les résultats sont comparés statistiquement (t test Student) par rapport à la valeur basale.
   ns : différence non significative par rapport à la valeur basale
   * : différence significative par rapport à la valeur basale, p<0,05
   ** : différence significative par rapport à la valeur basale, p<0,01
**Figure 9 :** Mise en évidence de l'existence d'un tonus vasculaire anormal et d'une vasorelaxation altérée chez la souris invalidée pour le gène CFTR (souris Cftr^{-/-)}
   **a,** tracés montrant la vasorelaxation induite par le neuropeptide VIP (Vasoactive Intestinal Polypeptide) d'aortes sans endothélium de souris Cftr^{+/+} (gauche) et Cftr^{-/-} (droite). Les anneaux d'aortes sont contractés reversiblement par une solution hyperpotassique (solution 80 mM K⁺) puis l'effet de 300 nM VIP est mesuré. L'absence de l'endothélium est vérifié par l'addition d'acetylcholine (ACh, 10⁻⁵ M).
   **b,** Histogrammes des tensions moyennes mesurées en réponse à une solution de 80 mM K⁺ pour des souris Cftr^{+/+} and Cftr^{-/-} avec (gauche) et sans (droit) endothélium.
   **C,** Histogrammes des moyennes de % de vasorelaxation induit par 300 nM VIP après constriction des anneaux d'aortes par 80 mM K⁺ pour des souris Cftr^{+/+} et Cftr^{-/-}.
   **d,** Courbes concentration-réponse pour le composé MPB-07 montrant une vasorelaxation d'anneaux d'aortes contractés par 80 mM K⁺ chez des souris Cftr^{+/+} (IC₅₀ + 37 ± 1.17 µM, n=6). Cet effet est absent chez des souris Cftr^{-/-} (n=6). Moyenne ± SEM* P < 0.05, **P < 0.01, *** P < 0.001.

### Bibliographie

DORMER, R.L., DERAND, R., MCNEILLY, C.M., METTEY, Y., BULTEAU-PIGNOUX, L., METAYE, T., VIERFOND, J.M., GRAY, M.A., GALIETTA, L.J., MORRIS, M.R., PEREIRA, M.M., DOULL, I.J., BECQ, F. & MCPHERSON, M.A. (2001). Correction of deIF508-CFTR activity with benzo(c)quinolizinium compounds through facilitation of its processing in cystic fibrosis airway cells. In *J Cell Sci.* pp. 4073-81.

BECQ, F., METTEY, Y., GRAY, M.A., GALIETTA, L.J., DORMER, R.L., MERTEN, M., METAYE, T., CHAPPE, V., MARVINGT-MOUNIR, C., ZEGARRA-MORAN, O., TARRAN, R., BULTEAU, L., DERAND, R., PEREIRA, M.M., MCPHERSON, M.A., ROGIER, C., JOFFRE, M., ARGENT, B.E., SARROUILHE, D., KAMMOUNI, W., FIGARELLA, C., VERRIER, B., GOLA, M. & VIERFOND, J.M. (1999). Development of substituted Benzo[c]quinolizinium compounds as novel activators of the cystic fibrosis chloride channel. *J Biol Chem,* **274,** 27415-25.

DERAND, R., BULTEAU-PIGNOUX, L., METTEY, Y., ZEGARRA-MORAN, O., HOWELL, L.D., RANDAK, C., GALIETTA, L.J., COHN, J.A., NOREZ, C., ROMIO, L., VIERFOND, J.M., JOFFRE, M. & BECQ, F. (2001). Activation of G551D CFTR channel with MPB-91: regulation by ATPase activity and phosphorylation. *Am J Physiol Cell Physiol,* **281**, C1657-66.

VENGLARIK, C.J., BRIDGES, R.J. & FRIZZELL, R.A. (1990). A simple assay for agonist-regulated Cl and K conductances in salt- secreting epithelial cells. *Am J Physiol,* **259**, C358-64.

## Revendications

1. Utilisation de dérivés de formule générale (I) suivante : 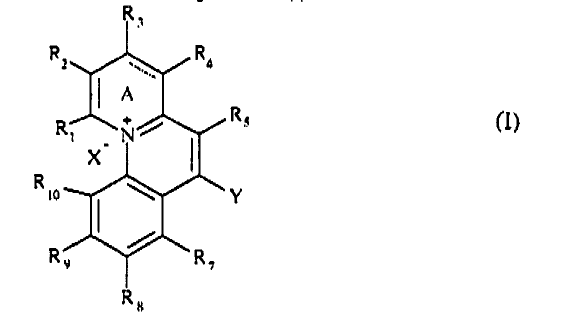 dans laquelle :
- l'hétérocycle A est aromatique ou non, étant entendu que dans ce dernier cas l'atome d'azote de cet hétérocycle est lié par une double liaison au carbone en position 4a,
- R₁, R₂, R₃, R₄, R₅, R₇, R₈, R₉ et R₁₀, représentent, indépendamment les uns des autres :
. un atome d'hydrogène, ou
. un atome d'halogène, notamment un atome de chlore, ou de brome, ou de fluor, ou
. un groupe alkyle, alkoxy, carbonyle, oxycarbonyle, ou ester, linéaire ou ramifié, d'environ 1 à environ 10 atomes de carbone, ces groupes étant le cas échéant substitués, notamment par un halogène, et/ou par un hydroxyle, et/ou par une amine (primaire, secondaire ou tertiaire), et/ou par un cycle aromatique et/ou aliphatique, d'environ 5 à environ 10 atomes de carbone dans le cycle, ces cycles étant eux-mérnes, le cas échéant, substitués notamment par un halogène, et/ou par un hydroxyle, et/ou par une amine (primaire, secondaire ou tertiaire), et/ou par un groupe alkyle, alkoxy, carbonyle, oxycarbonyle, ou ester, ces groupes étant tels que définis ci-dessus, ou
. un cycle aromatique ou aliphatique, d'environ 5 à environ 10 atomes de carbone dans le cycle, ce cycle étant lui-même, le cas échéant, substitué notamment par un halogène, et/ou par un hydroxyle, et/ou par une amine (primaire, secondaire ou tertiaire), et/ou par un groupe alkyle, alkoxy, carbonyle, oxycarbonyle, ou ester, ces groupes étant tels que définis ci-dessus, ou
. un groupe -ORₐ, Rₐ représentant un atome d'hydrogène, ou un groupe alkyle, carbonyle, oxycarbonyle, ou ester, linéaire ou ramifié, ces groupes étant tels que définis ci-dessus, ou un cycle aromatique ou aliphatique, ces cycles étant tels que définis ci-dessus, ou
. un groupe -NR_{b}R_{c}, R_{b} et R_{c}, indépendamment l'un de l'autre, représentant un atome d'hydrogène, un groupe alkyle, alkoxy, carbonyle, oxycarbonyle, ou ester, linéaire ou ramifié, ces groupes étant tels que définis ci-dessus, ou un cycle aromatique ou aliphatique, ces cycles étant tels que définis ci-dessus, ou
. lorsque R₁ et R₂, et/ou R₃ et R₄, et/ou R₄ et R₅, et/ou R₇ et R₈, et/ou R₈ et R₉, et/ou R₉ et R₁₀, ne représentent pas les différents atomes ou groupes ou cycles mentionnés ci-dessus, alors R₁ en association avec R₂, et/ou R₂ en association avec R₃, et/ou R₃ en association avec R₄, et/ou R₄ en association avec R₅, et/ou R₇ en association avec R₈, et/ou R₈ en association avec R₉, et/ou R₉ en association avec R₁₀, forment respectivement avec C₁ et C₂, ou avec C₂ et C₃, ou avec C₃ et C₄, ou avec C₄, C₄ₐ et C₅, ou avec C₇ et C₈, ou avec C₈ et C₉, ou avec C₉ et C₁₀, un cycle aromatique ou aliphatique de 5 à 10 atomes de carbone, ce cycle étant le cas échéant substitué, notamment par un halogène, et/ou par un groupe alkyle, alkoxy, carbonyle, oxycarbonyle, ou ester, et/ou par un cycle aromatique ou aliphatique, ces groupes ou cycles étant tels que définis ci-dessus, ou
. lorsque R₃ et R₄ ne représentent pas les différents atomes ou groupes ou cycles mentionnés ci-dessus, alors R₃ en association avec R₄ forment un groupe indole de formule 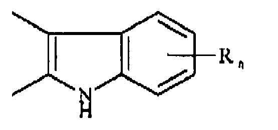
dans laquelle Rₐ est tel que défini ci-dessus,
- Y représente :
. un groupe -OR_{d}, R_{d} représentant un atome d'hydrogène, ou un groupe alkyle, carbonyle, oxycarbonyle, ou ester, linéaire ou ramifié, ces groupes étant tels que définis ci-dessus, ou un cycle aromatique ou aliphatique, ces cycles étant tels que définis ci-dessus, ou
. un groupe -NRₑR_{f}, Rₑ et R_{f} indépendamment l'un de l'autre, représentant un atome d'hydrogène, un groupe alkyle, alkoxy, carbonyle, oxycarbonyle, ou ester, linéaire ou ramifié, ces groupes étant tels que définis ci-dessus, ou un cycle aromatique ou aliphatique, ces cycles étant tels que définis ci-dessus,
. un groupe -SH,
. étant entendu que lorsque R_{d}, ou l'un au moins de R_{c} ou de R_{f}, ne représentent pas l'un des différents atomes ou groupes ou cycles mentionnés ci-dessus, alors R_{d}, ou l'un au moins de Rₑ ou de R_{f}, en association avec R₅, ou en association avec R₇, forment respectivement avec C₅ et C₆, ou avec C₆, C₆ₐ et C₇. un hétérocycle aromatique ou aliphatique de 5 à 1 () atomes de carbone, le cas échéant substitué, notamment par un halogène, et/ou par un groupe alkyle, alkoxy, carbonyle, oxycarbonyle, ou ester, et/ou par un cycle aromatique ou aliphatique, ces groupes ou cycles étant tels que définis ci-dessus,
- X représente un atome sous forme anionique, tel qu'un atome d'halogène, notamment un atome de brome ou de chlore, ou un groupe d'atomes sous forme anionique, tel qu'un perchlorate, et l'azote de l'hétérocycle A de la formule (I) est sous forme quaternaire et est lié d'une part par liaison covalente au carbone en position 11, et, d'autre part, par liaison ionique à X défini ci-dessus, étant entendu que lorsque R₁ et R₁₀ ne représentent pas l'un des différents atomes ou groupes ou cycles mentionnés ci-dessus, alors R₁ en association avec R₁₀ forment avec C₁, l'azote de l'hétérocycle A de la formule (I), C₁₁, et C₁₀, un hétérocycle aromatique ou aliphatique de 5 à 10 atomes de carbone, le cas échéant substitué, notamment par un halogène, et/ou par un groupe alkyle, alkoxy, carbonyle, oxycarbonyle, ou ester, et/ou par un cycle aromatique ou aliphatique, ces groupes ou cycles étant tels que définis ci-dessus,
pour la préparation de médicaments destinés au traitement de pathologies liées une constriction des cellules musculaires lisses dans les tissus telles que les pathologies liées aux phénomènes de vasoconstriction dans le cadre de troubles vasculaires, notamment l'hypertension artérielle, ou les pathologies liées aux phénomènes de bronchoconstriction dans le cadre de troubles de la respiration, notamment l'asthme.

2. Utilisation selon la revendication 1, des dérivés des benzo[c] quinoliziniums de formule (Ia) suivante : 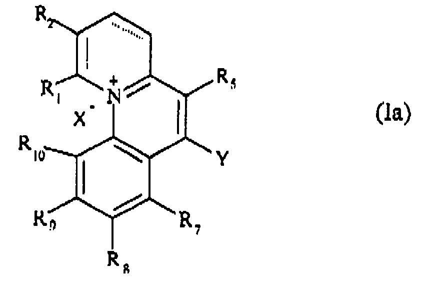 dans laquelle :
- R₁ et R₂ représentent un atome d'hydrogène, ou forment en association avec C₁ et C₂ un cycle aromatique à 6 atomes de carbone,
- R₅ représente un atome d'hydrogène, ou un groupe alkyle linéaire ou substitué de 1 à 10 atomes de carbone, notamment un groupe butyle, ou un ester de formule COOR' dans laquelle R' représente un groupe alkyle linéaire ou substitué de 1 à 10 atomes de carbone, notamment un groupe éthyle,
- Y représente un groupe -OH, -SH, -NH₂, ou -NHCOCH₃,
- R₇, R₈, R₉ et R₁₀ représentent un atome d'hydrogène, ou l'un au moins de R₇, R₈, R₉ ou R₁₀, représente un atome d'halogène, notamment un atome de chlore, de brome ou de fluor,
- X représente un atome d'halogène sous forme anionique, notamment un atome de brome Br⁻, ou de chlore Cl⁻, ou un groupe d'atomes sous forme anionique.

3. Utilisation selon la revendication 1 ou 2. des dérivés des benzo[c] quinoliziniums de formule (Ia) dans laquelle Y représente un groupe -NH₂, ou -NHCOCH₃.

4. Utilisation selon la revendication 3, des dérivés des benzo[c]quinoliziniums de formule (Ia) suivants :

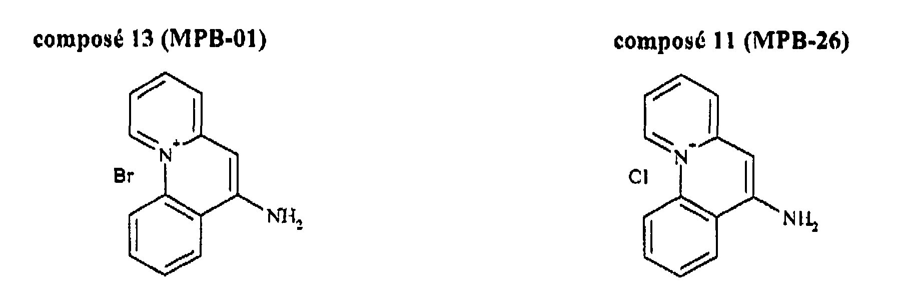

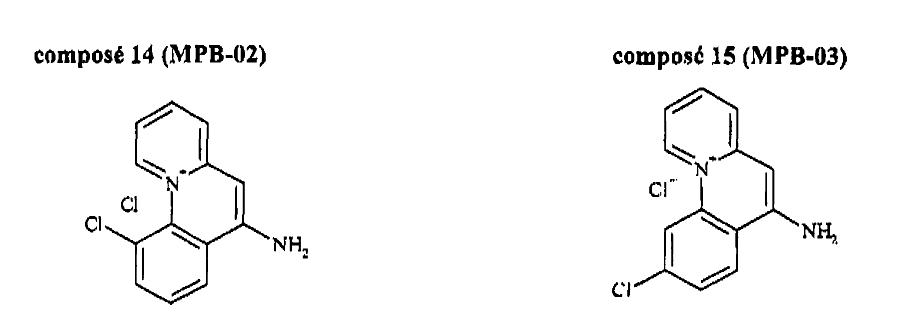

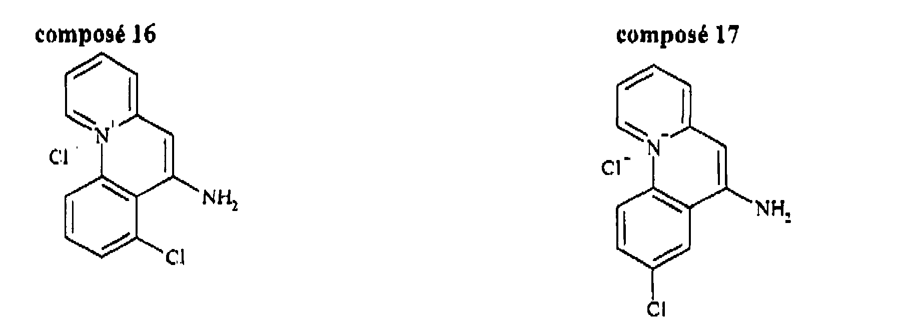

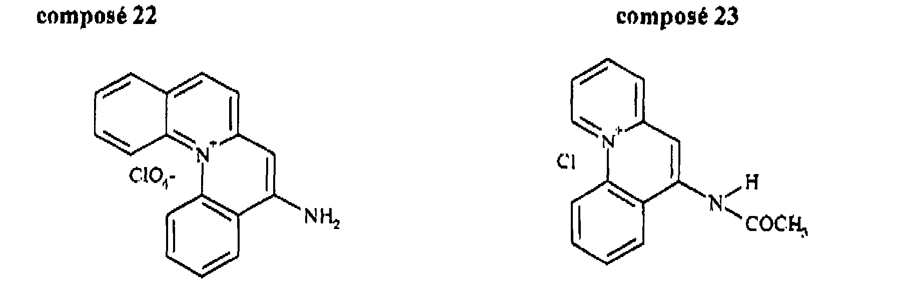

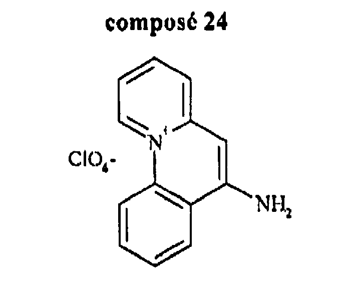

5. Utilisation selon la revendication 1 ou 2, de dérivés des benzo[c]quinoliziniums de formule (Ia) dans laquelle Y représente OH.

6. Utilisation selon la revendication 5, de dérivés des benzo[c]quinoliziniums de formule (Ia) choisis parmi les suivants ;

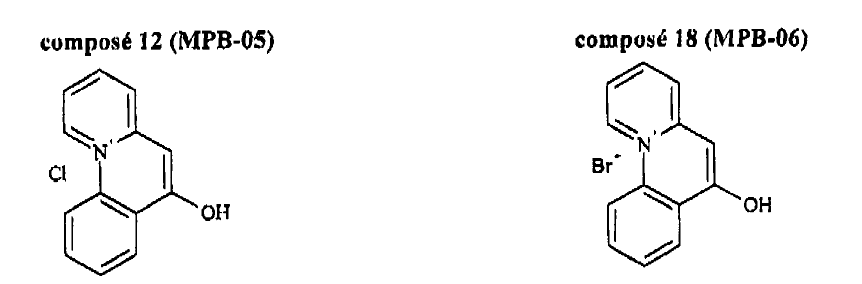

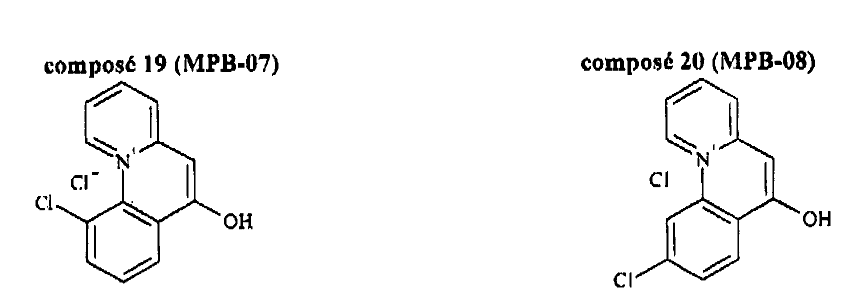

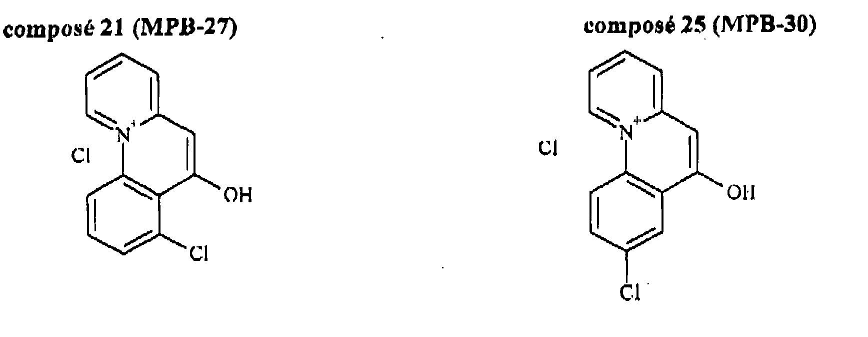

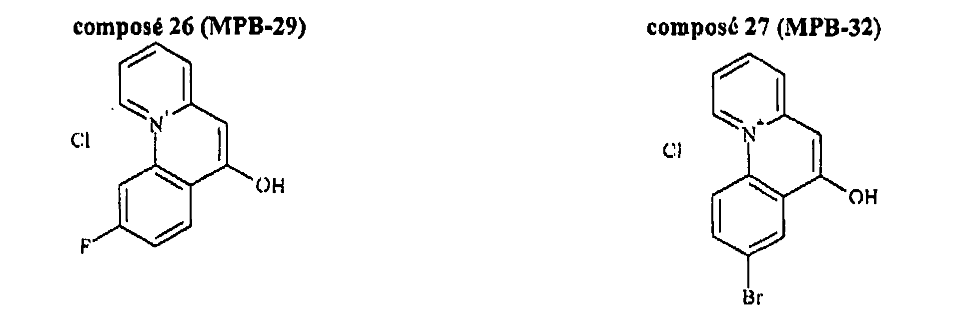

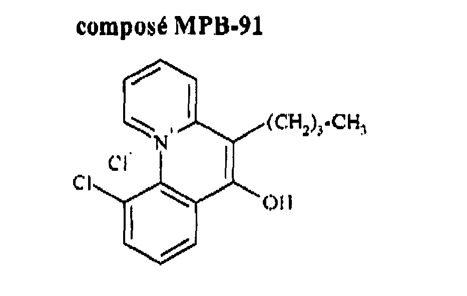

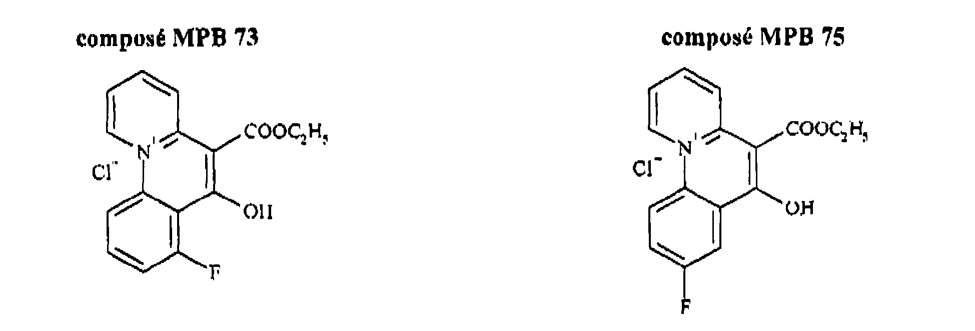

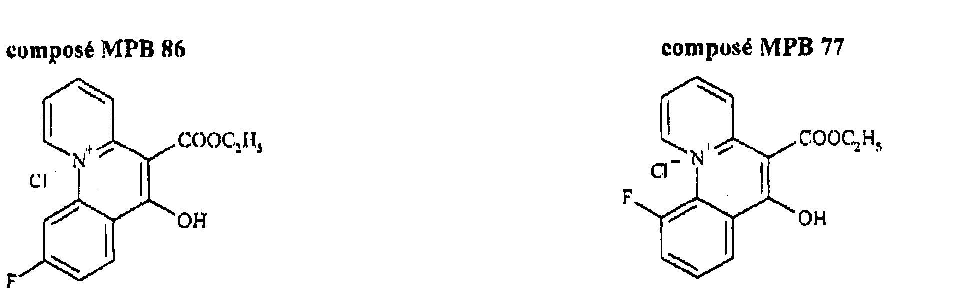

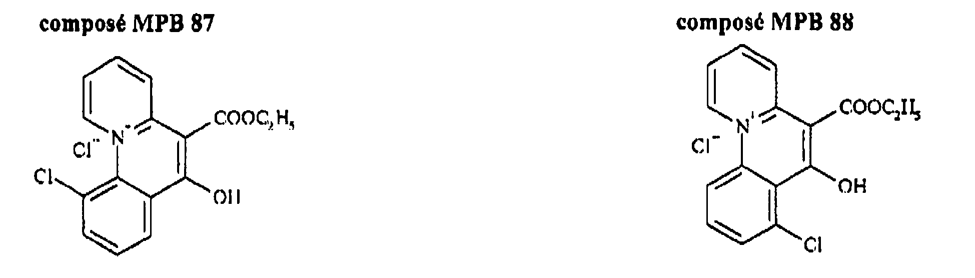

7. Utilisation selon la revendication 1 ou 2, de dérivés des benzo[c]quinoliziniums de formule (Ia) dans laquelle Y représente SH.

8. Utilisation selon la revendication 7, de dérivés des benzo[c]quinoliziniums de formule (Ia) choisis parmi les suivants ;

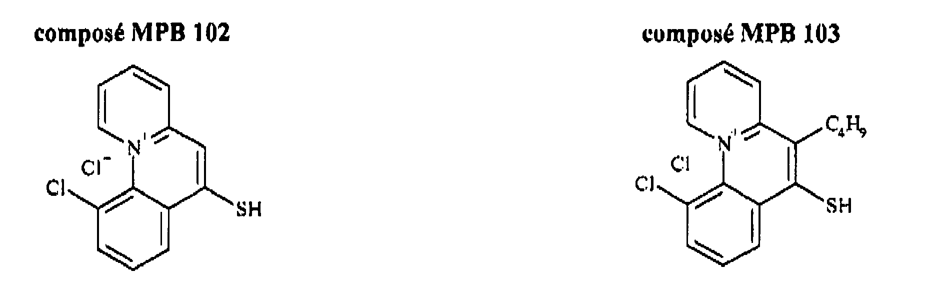

9. Utilisation selon la revendication 5, de dérivés de formule générale (Ia-1) suivante : 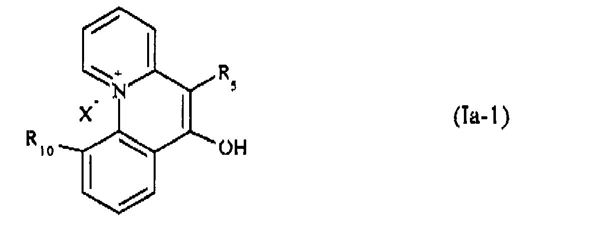 dans laquelle :
- R₅ représente un atome d'hydrogène, ou un groupe alkyle linéaire ou substitué de 1 à 10 atomes de carbone, notamment un groupe butyle,
- R₁₀ représente un atome un atome d'halogène, notamment un atome de chlore, de brome ou de fluor,
- X représente un atome d'halogène sous forme anionique, notamment un atome de brome Br⁻, ou de chlore Cl⁻, ou un groupe d'atomes sous forme anionique, notamment un perchlorate ClO₄⁻,

10. Utilisation selon la revendication 9, du dérivé MPB-07 de formule suivante :

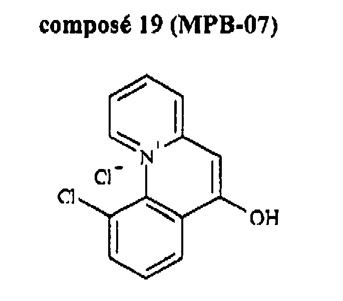

11. Utilisation selon la revendication 9, du dérivé MPB-91 de formule suivante :

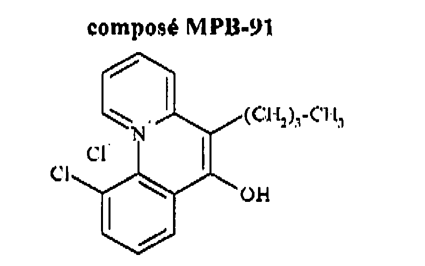

12. Utilisation selon la revendication 1, de dérivés de formule générale (Ib) suivante : 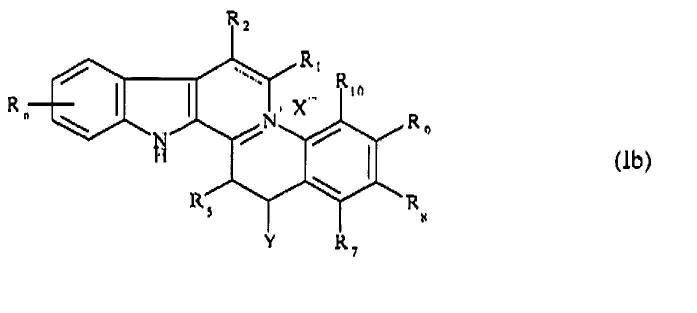 dans laquelle Rₐ, R₁, R₂, R₅, R₇, R₈, R₉, R₁₀, X et Y sont tels que définis dans la revendication 1, et notamment les composés de formule (Ib) dans laquelle:
- Rₐ représente un atome d'hydrogène.
- R₁ et R₂ représentent un atome d'hydrogène, et il n'y a pas de double liaison entre les deux carbones portant R₁ et R₂,
- R₅ représente un atome d'hydrogène,
- R₇, R₈. R₉ et R₁₀ représentent un atome d'hydrogène, ou l'un de R₇, R₈, R₉ ou R₁₀ représente un atome d'halogène, notamment un atome de chlore, de brome ou de fluor,
- Y représente -NH₂ ou -OH,
- X représente un atome d'halogène, notamment un atome de brome, ou de chlore, ou de fluor.

13. Utilisation selon la revendication 10, de dérivés de formule (Ib-1) suivante : 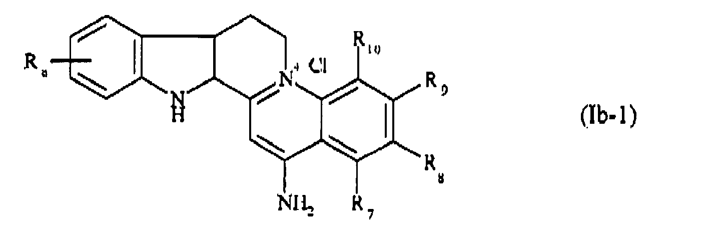 et plus particulièrement les composés de formule (1b-1) suivants :
- composé G R₇ = Cl, R₈ = R₉ = R₁₀ = H,
- composé H : R₇ = R₈ - R₉ - R₁₀ - H,
- composé I : R₈=Cl, R₇ = R₉= R₁₀= H,
- composé J : R₉ = Cl, R₇ = R₈ = R₁₀ = H,
- composé K : R₁₀ = Cl, R₇ = R₈ = R₉ = H,
- composé L : R₉ - Br, R₇ - R₈ - R₁₀ = H.

14. Composés de formule (1) telle que définie dans la revendication 1 dans laquelle R₅ représente un ester de formule COOR' dans laquelle R' représente un groupe alkyle linéaire ou substitué de 1 à 10 atomes de carbone, notamment un groupe éthyle,

15. Composés selon la revendication 14, de formule (Ia) telle que définie dans la revendication 2, dans laquelle :
- R₁ et R₂ représentent un atome d'hydrogène, ou forment en association avec C₁ et C₂ un cycle aromatique à 6 atomes de carbone,
- R₅ représente un ester de formule COOR' dans laquelle R' représente un groupe alkyle linéaire ou substitué de 1 à 10 atomes de carbone, notamment un groupe éthyle,
- Y représente un groupe -OH, -SH, -NH₂, ou -NHCOCH₃,
- R₇, R₈, R₉ et R₁₀ représentent un atome d'hydrogène, ou l'un au moins de R₇, R₈, R₉ ou R₁₀, représente un atome d'halogène, notamment un atome de chlore, de brome ou de fluor,
- X représente un atome d'halogène sous forme anionique, notamment un atome de brome Br⁻, ou de chlore Cl⁻, ou un groupe d'atomes sous forme anionique.

16. Composés selon la revendication 14 ou 15, de formule (la) dans laquelle R₅ représente un ester de formule COOR' dans laquelle R' représente un groupe alkyle linéaire ou substitué de 1 à 10 atomes de carbone, notamment un groupe éthyle, et Y représente un groupe -OH.

17. Composés selon l'une des revendications 14 à 16, de formules suivantes:

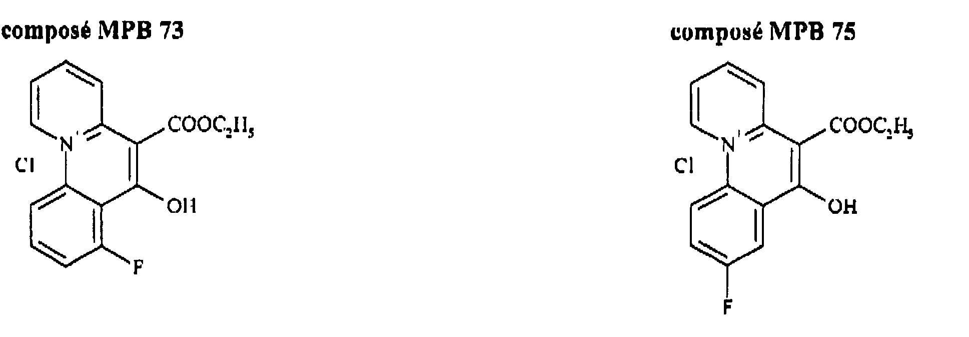

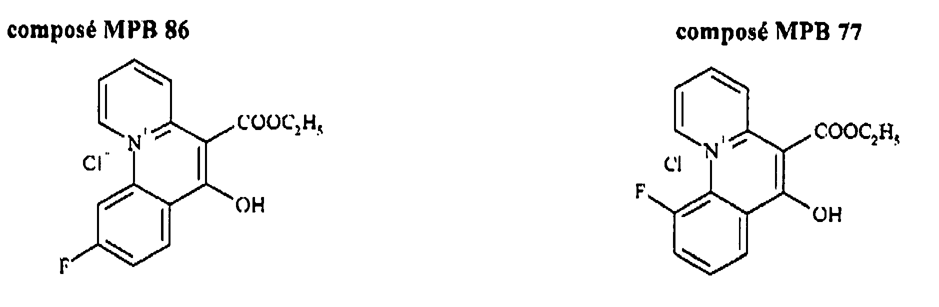

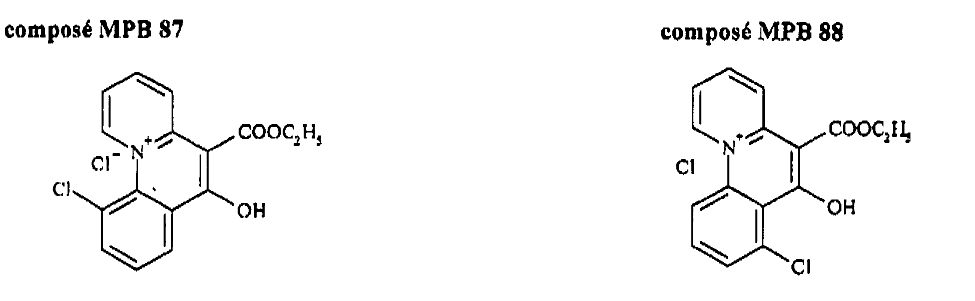

18. Composés de formule (I) telle que définie dans la revendication 1 dans laquelle Y représente SH.

19. Composés selon la revendication 18, de formule (Ia) telle que définie dans la revendication 2, dans laquelle :
- R₁ et R₂ représentent un atome d'hydrogène, ou forment en association avec C₁ et C₂ un cycle aromatique à 6 atomes de carbone,
- R₅ représente un atome d'hydrogène, ou un groupe alkyle linéaire ou substitué de 1 à 10 atomes de carbone, notamment un groupe butyle.
- Y représente un groupe -SH,
- R₇, R₈, R₉ et R₁₀ représentent un atome d'hydrogène, ou l'un au moins de R₇, R₈, R₉ ou R₁₀, représente un atome d'halogène, notamment un atome de chlore, de brome ou de fluor,
- X représente un atome d'halogène sous forme anionique, notamment un atome de brome Br⁻, ou de chlore Cl⁻, ou un groupe d'atomes sous forme anionique.

20. Composés selon la revendication 18 ou 19, de formules suivantes:

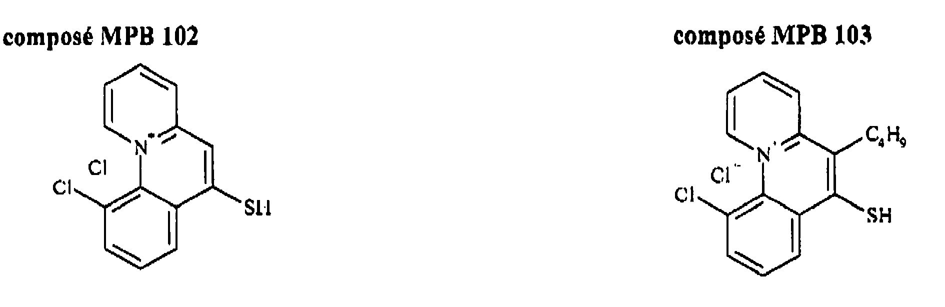

21. Compositions pharmaceutiques comprenant au moins un composé défini dans l'une des revendications 14 à 20, en association avec un véhicule pharmaceutiquement acceptable.

## Claims

1. Use of derivatives of the following general formula (I): 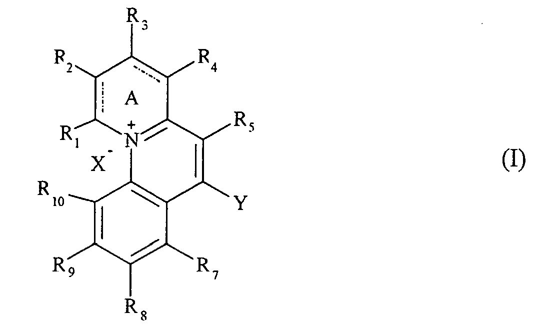 in which:
- the heterocycle A is aromatic or non-aromatic, it being understood that in this latter case the nitrogen atom of this heterocycle is linked by a double bond to the carbon in position 4a,
- R₁, R₂, R₃, R₄, R₅, R₇, R₈, R₉ and R₁₀, represent, independently of one another:
. a hydrogen atom, or
. a halogen atom, in particular a chlorine, bromine, or fluorine atom, or
. an alkyl, alkoxy, carbonyl, oxycarbonyl or ester group, linear or branched, with approximately 1 to approximately 10 carbon atoms, these groups being if appropriate substituted, in particular by a halogen, and/or by a hydroxyl, and/or by a (primary, secondary or tertiary) amine, and/or by an aromatic and/or aliphatic ring, with approximately 5 to approximately 10 carbon atoms in the ring, these rings being themselves, if appropriate, substituted in particular by a halogen, and/or by a hydroxyl, and/or by a (primary, secondary or tertiary) amine, and/or by an alkyl, alkoxy, carbonyl, oxycarbonyl or ester group, these groups being as defined above, or
. an aromatic or aliphatic ring, with approximately 5 to approximately 10 carbon atoms in the ring, this ring being itself, if appropriate, substituted in particular by a halogen, and/or by a hydroxyl, and/or by a (primary, secondary or tertiary) amine, and/or by an alkyl, alkoxy, carbonyl, oxycarbonyl or ester group, these groups being as defined above, or
. an -ORₐ group, Rₐ representing a hydrogen atom, or an alkyl, carbonyl, oxycarbonyl or ester group, linear or branched, these groups being as defined above, or an aromatic or aliphatic ring, these rings being as defined above, or
. an -NR_{b}R_{c} group, R_{b} and R_{c}, independently of one another, representing a hydrogen atom, an alkyl, alkoxy, carbonyl, oxycarbonyl or ester group, linear or branched, these groups being as defined above, or an aromatic or aliphatic ring, these rings being as defined above, or
. when R₁ and R₂, and/or R₃ and R₄, and/or R₄ and R₅, and/or R₇ and R₈, and/or R₈ and R₉, and/or R₉ and R₁₀, do not represent the different atoms or groups or rings mentioned above, then R₁ in combination with R₂, and/or R₂ in combination with R₃, and/or R₃ in combination with R₄, and/or R₄ in combination with R₅, and/or R₇ in combination with R₈, and/or R₈ in combination with R₉, and/or R₉ in combination with R₁₀, respectively form with C₁ and C₂, or with C₂ and C₃, or with C₃ and C₄, or with C₄, C₄ₐ and C₅, or with C₇ and C₈, or with C₈ and C₉, or with C₉ and C₁₀, an aromatic or aliphatic ring with 5 to 10 carbon atoms, this ring being if appropriate substituted, in particular by a halogen, and/or by an alkyl, alkoxy, carbonyl, oxycarbonyl, or ester group, and/or by an aromatic or aliphatic ring, these groups or rings being as defined above, or
. when R₃ and R₄ do not represent the different atoms or groups or rings mentioned above, then R₃ in combination with R₄ forms an indole group of formula 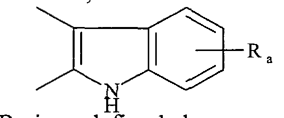
in which Rₐ is as defined above,
- Y represents:
. an -OR_{d} group, R_{d} representing a hydrogen atom, or an alkyl, carbonyl, oxycarbonyl or ester group, linear or branched, these groups being as defined above, or an aromatic or aliphatic ring, these rings being as defined above, or
. an -NRₑR_{f} group, Rₑ and R_{f} independently of one another, representing a hydrogen atom, or an alkyl, alkoxy, carbonyl, oxycarbonyl or ester group, linear or branched, these groups being as defined above, or an aromatic or aliphatic ring, these rings being as defined above,
. an -SH group,
. it being understood that when R_{d}, or at least one of Rₑ or R_{f}, do not represent one of the different atoms or groups or rings mentioned above, then R_{d}, or at least one of Rₑ or R_{f}, in combination with R₅, or in combination with R₇, respectively form with C₅ and C₆, or with C₆, C₆ₐ and C₇, an aromatic or aliphatic heterocycle with 5 to 10 carbon atoms, if appropriate substituted, in particular by a halogen, and/or by an alkyl, alkoxy, carbonyl, oxycarbonyl or ester group, and/or by an aromatic or aliphatic ring, these groups or rings being as defined above,
- X represents an atom in anionic form, such as a halogen atom, in particular a bromine or chlorine atom, or a group of atoms in anionic form, such as a perchlorate, and the nitrogen of the heterocycle A of formula (I) is in quaternary form and is linked on the one hand by a covalent bond to the carbon in position 11, and, on the other hand, by ionic bond to X defined above, it being understood that when R₁ and R₁₀ do not represent one of the different atoms or groups or rings mentioned above, then R₁ in combination with R₁₀ forms with C₁, the nitrogen of the heterocycle A of formula (I), C₁₁, and C₁₀, an aromatic or aliphatic heterocycle with 5 to 10 carbon atoms, if appropriate substituted, in particular by a halogen, and/or by an alkyl, alkoxy, carbonyl, oxycarbonyl or ester group, and/or by an aromatic or aliphatic ring, these groups or rings being as defined above,
for the preparation of medicaments intended for the treatment of pathologies linked to a constriction of smooth muscle cells in tissues such as the pathologies linked to vasoconstriction phenomena within the scope of vascular disorders, in particular arterial hypertension, or the pathologies linked to bronchoconstriction phenomena within the scope of respiratory disorders, in particular asthma.

2. Use according to claim 1, of the derivatives of benzo[c] quinoliziniums of following formula (Ia): 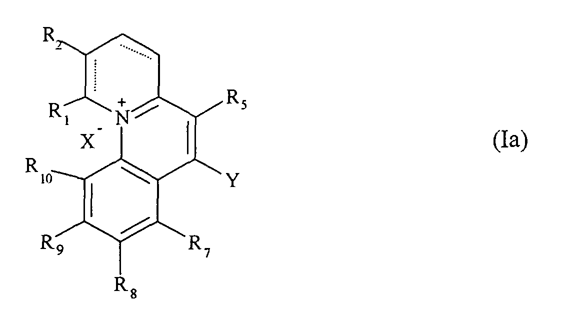 in which:
- R₁ and R₂ represent a hydrogen atom, or form in combination with C₁ and C₂ an aromatic ring with 6 carbon atoms,
- R₅ represents a hydrogen atom, or a linear or substituted alkyl group with 1 to 10 carbon atoms, in particular a butyl group, or an ester of formula COOR' in which R' represents a linear or substituted alkyl group with 1 to 10 carbon atoms, in particular an ethyl group,
- Y represents an -OH, -SH, -NH₂, or -NHCOCH₃ group,
- R₇, R₈, R₉ and R₁₀ represent a hydrogen atom, or at least one of R₇, R₈, R₉ or R₁₀, represents a halogen atom, in particular a chlorine, bromine or fluorine atom,
- X represents a halogen atom in anionic form, in particular a bromine Br⁻, or chlorine Cl- atom, or a group of atoms in anionic form.

3. Use according to claim 1 or 2, of the derivatives of benzo[c] quinoliziniums of formula (Ia) in which Y represents an -NH₂, or -NHCOCH₃ group.

4. Use according to claim 3, of the following derivatives of benzo[c]quinoliziniums of formula (Ia):

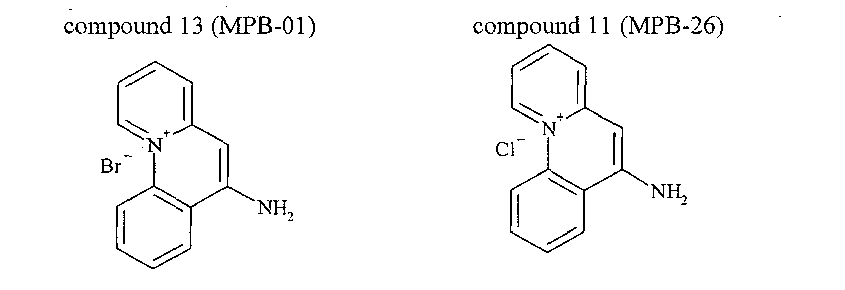

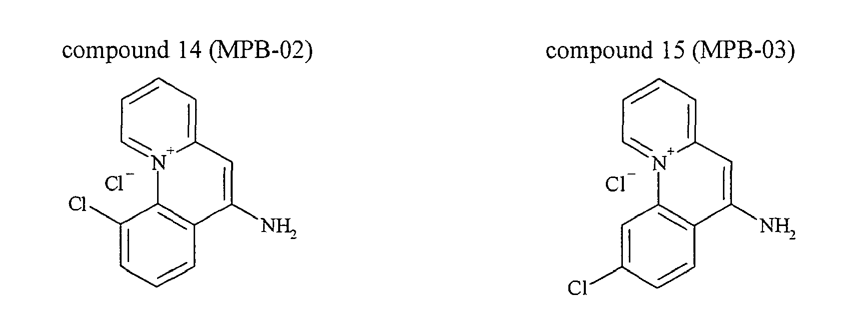

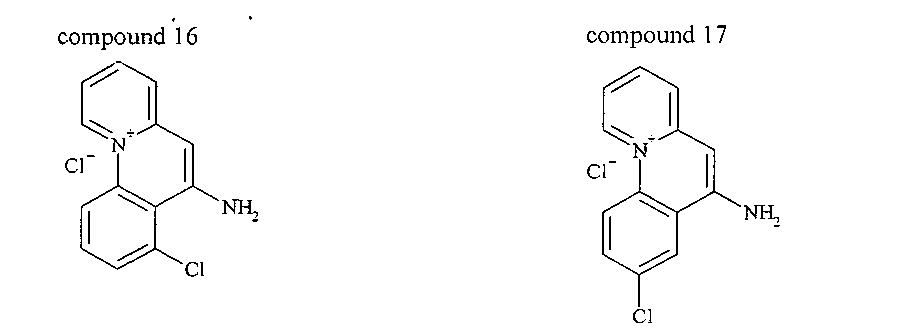

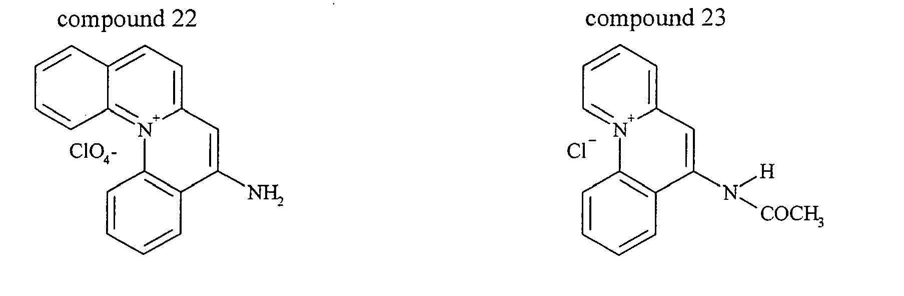

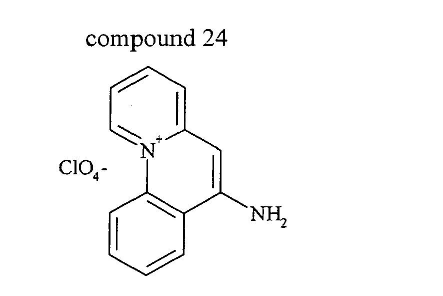

5. Use according to claim 1 or 2, of derivatives of the benzo[c]quinoliziniums of formula (Ia) in which Y represents OH.

6. Use according to claim 5, of derivatives of the benzo[c]quinoliziniums of formula (Ia) chosen from the following:

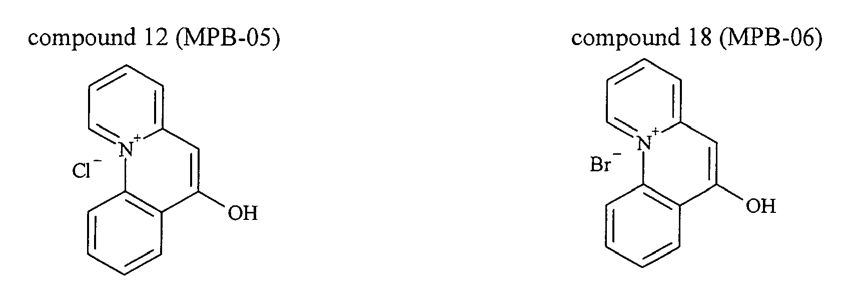

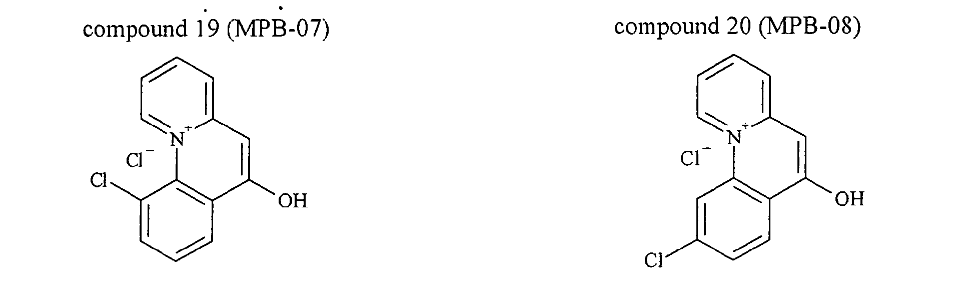

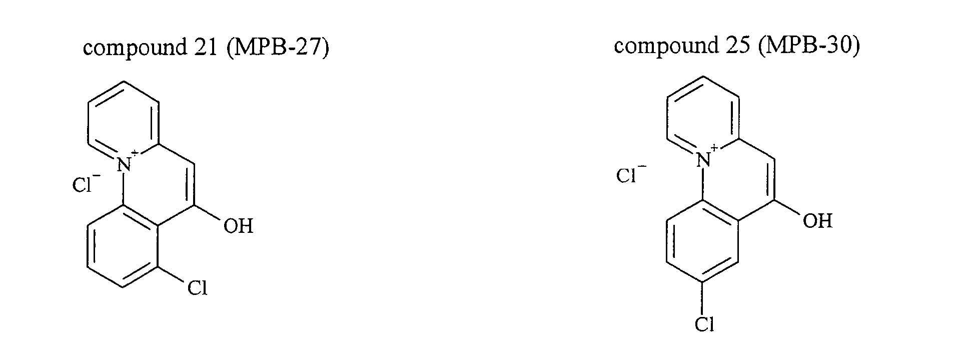

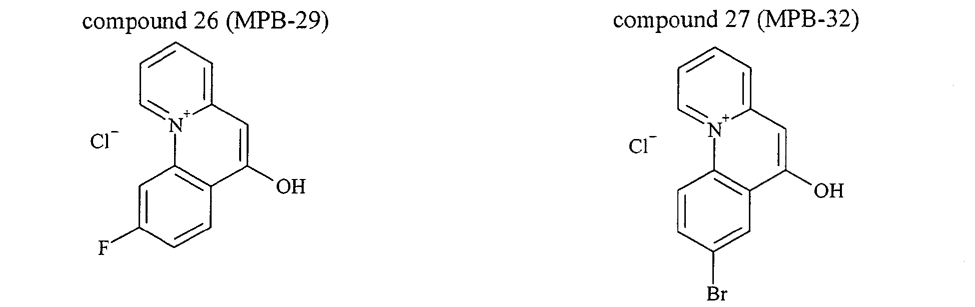

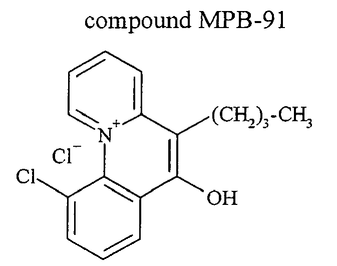

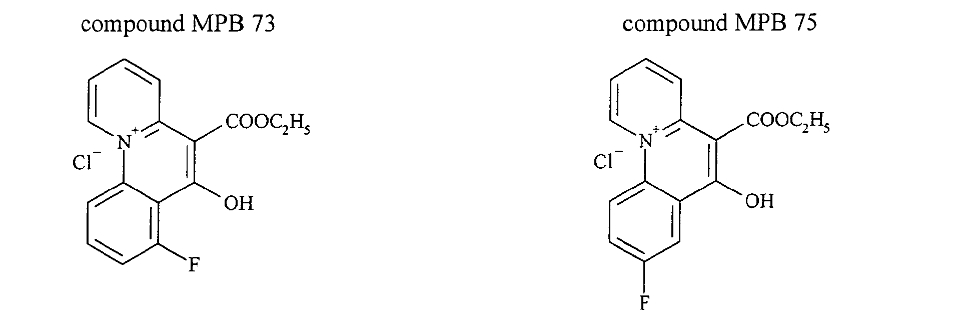

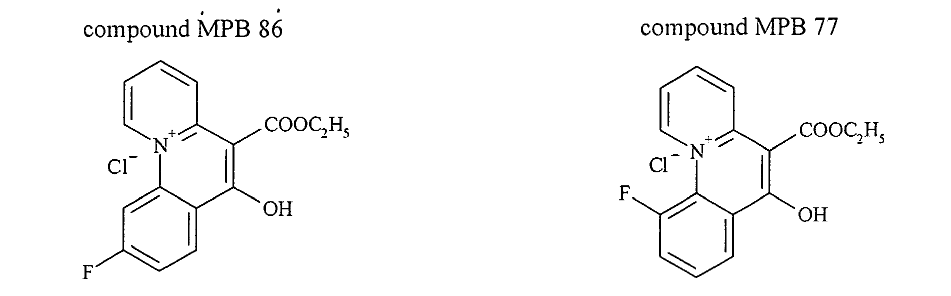

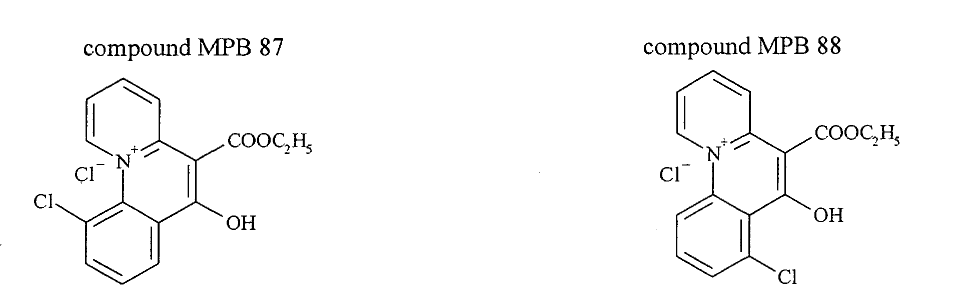

7. Use according to claim 1 or 2, of derivatives of the benzo[c]quinoliziniums of formula (Ia) in which Y represents SH.

8. Use according to claim 7, of derivatives of the benzo[c]quinoliziniums of formula (Ia) chosen from the following:

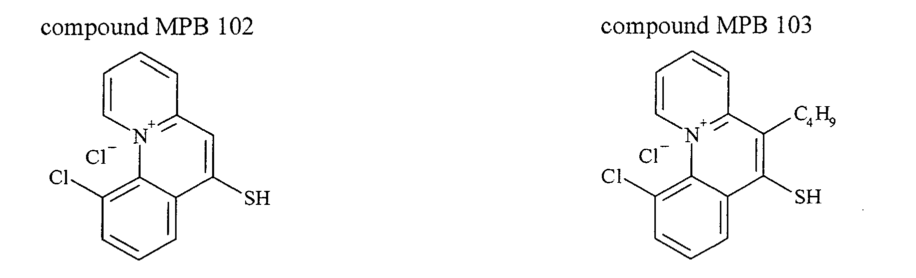

9. Use according to claim 5, of derivatives of the following general formula (Ia-1): 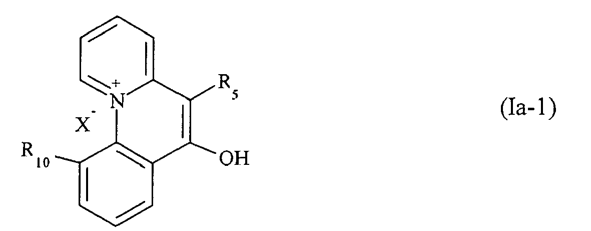 in which:
- R₅ represents a hydrogen atom, or a linear or substituted alkyl group with 1 to 10 carbon atoms, in particular a butyl group,
- R₁₀ represents a halogen atom, in particular a chlorine, bromine or fluorine atom,
- X represents a halogen atom in anionic form, in particular a bromine Br⁻ or chlorine Cl- atom, or a group of atoms in anionic form, in particular a perchlorate ClO₄⁻.

10. Use according to claim 9, of the derivative MPB-07 of following formula:

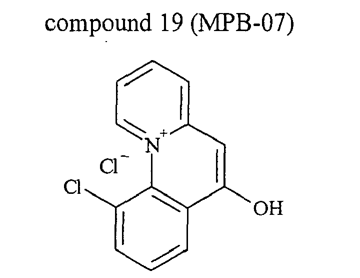

11. Use according to claim 9, of the derivative MPB-91 of following formula:

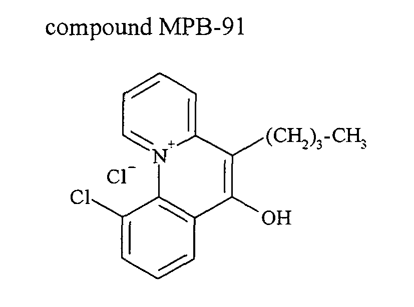

12. Use according to claim 1, of derivatives of following general formula (Ib): 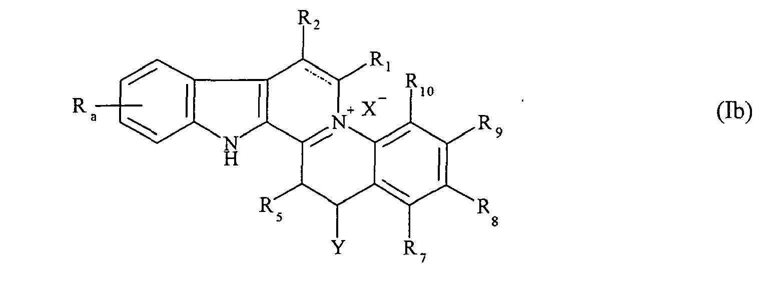 in which Rₐ, R₁, R₂, R₅, R₇, R₈, R₉, R₁₀, X and Y are as defined in claim 1, and in particular the compounds of formula (Ib) in which:
- Rₐ represents a hydrogen atom,
- R₁ and R₂ represent a hydrogen atom, and there is no double bond between the two carbons carrying R₁ and R₂,
- R₅ represents a hydrogen atom,
- R₇, R₈, R₉ and R₁₀ represent a hydrogen atom, or one of R₇, R₈, R₉ or R₁₀ represents a halogen atom, in particular a chlorine, bromine or fluorine atom,
- Y represents NH₂ or OH,
- X represents a halogen atom, in particular a bromine, or chlorine, or fluorine atom.

13. Use according to claim 10, of derivatives of following formula (Ib-1): 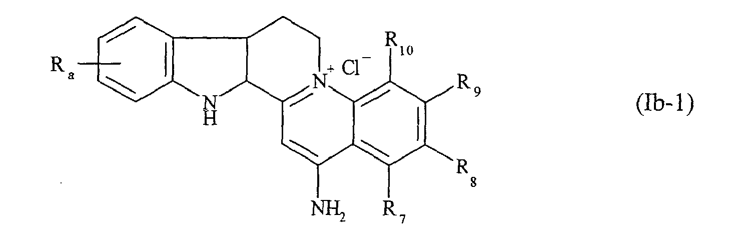 and more particularly the following compounds of formula (Ib-1):
- compound G: R₇ = Cl, R₈ = R₉ = R₁₀= H,
- compound H: R₇ = R₈ = R₉ = R₁₀ = H,
- compound 1: R₈ = Cl, R₇ = R₉ = R₁₀ = H,
- compound J: R₉ = Cl, R₇ R₈ = R₁₀ = H,
- compound K: R₁₀ = Cl, R₇ = R₈ = R₉ = H,
- compound L: R₉ = Br, R₇ = R₈ = R₁₀ = H.

14. Compounds of formula (I) as defined in claim 1 in which R₅ represents an ester of formula COOR' in which R' represents a linear or substituted alkyl group with 1 to 10 carbon atoms, in particular an ethyl group.

15. Compounds according to claim 14, of formula (Ia) as defined in claim 2, in which:
- R₁ and R₂ represent a hydrogen atom, or form in combination with C₁ and C₂ an aromatic ring with 6 carbon atoms,
- R₅ represents an ester of formula COOR' in which R' represents a linear or substituted alkyl group with 1 to 10 carbon atoms, in particular an ethyl group,
- Y represents an -OH, -SH, -NN₂, or -NHCOCH₃ group,
- R₇, R₈, R₉ and R₁₀ represent a hydrogen atom, or at least one of R₇, R₈, R₉ or R₁₀ represents a halogen atom, in particular a chlorine, bromine or fluorine atom,
- X represents a halogen atom in anionic form, in particular a bromine Br-, or chlorine Cl- atom, or a group of atoms in anionic form.

16. Compounds according to claim 14 or 15, of formula (Ia) in which R₅ represents an ester of formula COOR' in which R' represents a linear or substituted alkyl group with 1 to 10 carbon atoms, in particular an ethyl group, and Y represents an -OH group.

17. Compounds according to one of claims 14 to 16, of following formulae:

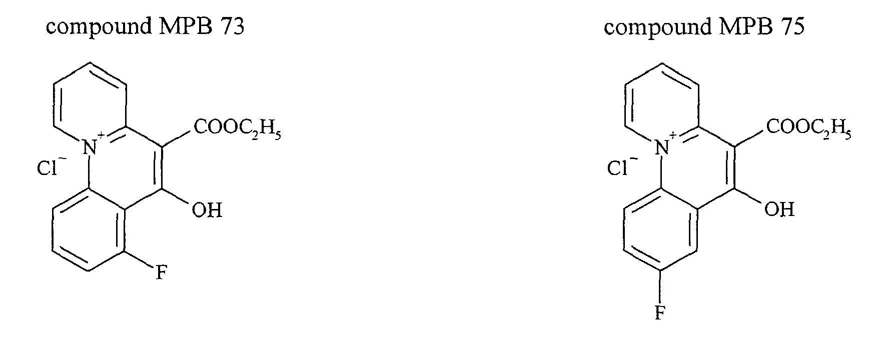

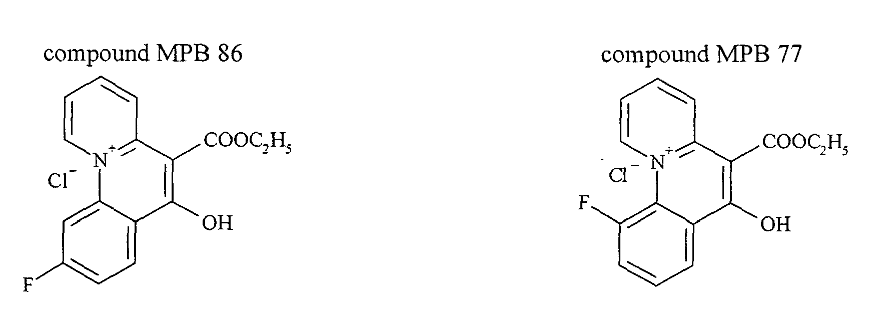

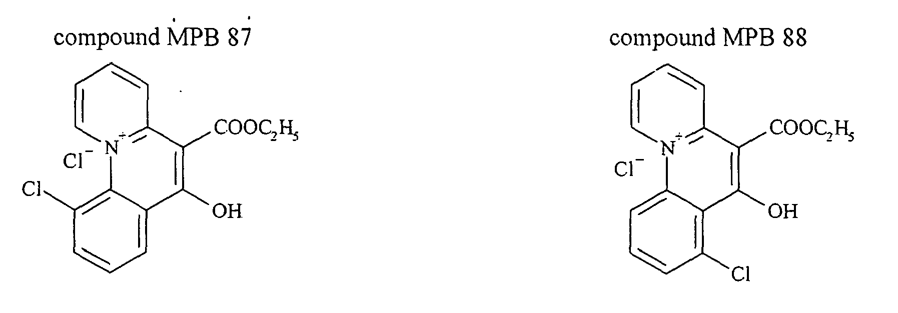

18. Compounds of formula (I) as defined in claim 1 in which Y represents SH.

19. Compounds according to claim 18, of formula (Ia) as defined in claim 2, in which:
- R₁ and R₂ represent a hydrogen atom, or form in combination with C₁ and C₂ an aromatic ring with 6 carbon atoms,
- R₅ represents a hydrogen atom, or a linear or substituted alkyl group with 1 to 10 carbon atoms, in particular a butyl group,
- Y represents an -SH group,
- R₇, R₈, R₉ and R₁₀ represent a hydrogen atom, or at least one of R₇, R₈, R₉ or R₁₀ represents a halogen atom, in particular a chlorine, bromine or fluorine atom,
- X represents a halogen atom in anionic form, in particular a bromine Br⁻, or chlorine Cl⁻ atom, or a group of atoms in anionic form.

20. Compounds according to claim 18 or 19, of following formulae:

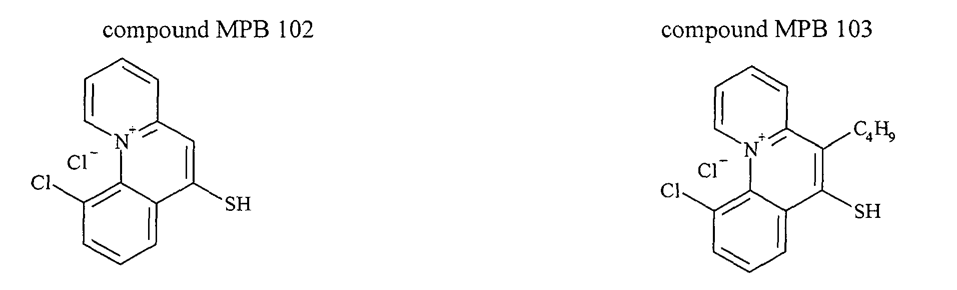

21. Pharmaceutical compositions comprising at least one compound defined in one of claims 14 to 20, in combination with a pharmaceutically acceptable vehicle.

## Patentansprüche

1. Verwendung von Derivaten der folgenden allgemeinen Formel (I) 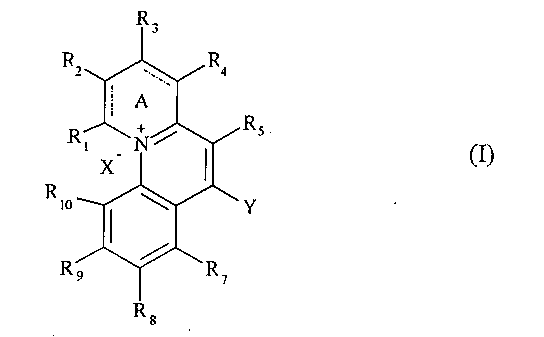 worin:
- der Heterocyclus A aromatisch oder nicht aromatisch ist, mit der Maßgabe, dass im letzteren Fall das Stickstoffatom dieses Heterocyclus durch eine Doppelbindung an den Kohlenstoff in der Position 4a gebunden ist,
- R₁, R₂, R₃, R₄, R₅, R₇, R₈, R₉ und R₁₀ unabhängig voneinander repräsentieren:
· ein Wasserstoffatom oder
· ein Halogenatom, insbesondere ein Chlor- oder Brom- oder Fluoratom, oder
• eine Alkyl-, Alkoxy-, Carbonyl-, Oxycarbonyl- oder Estergruppe, linear oder verzweigt, mit etwa 1 bis etwa 10 Kohlenstoffatomen, wobei diese Gruppen gegebenenfalls substituiert sind, insbesondere mit einem Halogen und/oder Hydroxyl und/oder Amin (primär, sekundär oder tertiär) und/oder einem aromatischen und/oder aliphatischen Ring mit etwa 5 bis etwa 10 Kohlenstoffatomen im Ring, wobei diese Ringe ihrerseits gegebenenfalls substituiert sind, insbesondere mit einem Halogen und/oder Hydroxyl und/oder Amin (primär, sekundär oder tertiär) und/oder einer Alkyl-, Alkoxy-, Carbonyl-, Oxycarbonyl- oder Estergruppe, wobei diese Gruppen wie oben definiert sind, oder
• einen aromatischen oder aliphatischen Ring mit etwa 5 bis etwa 10 Kohlenstoffatomen im Ring, wobei der Ring seinerseits gegebenenfalls substituiert ist, insbesondere mit einem Halogen und/oder Hydroxyl und/oder Amin (primär, sekundär oder tertiär) und/oder einer Alkyl-, Alkoxy-, Carbonyl-, Oxycarbonyl- oder Estergruppe, wobei diese Gruppen wie oben definiert sind, oder
• eine Gruppe -ORₐ, wobei Rₐ ein Wasserstoffatom oder eine Alkyl-, Carbonyl-, Oxycarbonyl- oder Estergruppe, linear oder verzweigt, wobei diese Gruppen wie oben definiert sind, oder einen aromatischen oder aliphatischen Ring, wobei diese Ringe wie oben definiert sind, darstellt, oder
• eine Gruppe -NR_{b}R_{c}, wobei R_{b} und R_{c} unabhängig voneinander ein Wasserstoffatom, eine Alkyl-, Alkoxy-, Carbonyl-, Oxycarbonyl- oder Estergruppe, linear oder verzweigt, wobei diese Gruppen wie oben definiert sind, oder einen aromatischen oder aliphatischen Ring, wobei diese Ringe wie oben definiert sind, darstellen, oder
• wenn R₁ und R₂ und/oder R₃ und R₄ und/oder R₄ und R₅ und/oder R₇ und R₈ und/oder R₈ und R₉ und/oder R₉ und R₁₀ nicht die verschiedenen oben genannten Atome oder Gruppen oder Ringe repräsentieren, dann bildet R₁ gemeinsam mit R₂ und/oder R₂ gemeinsam mit R₃ und/oder R₃ gemeinsam mit R₄ und/oder R₄ gemeinsam mit R₅ und/oder R₇ gemeinsam mit R₈ und/oder R₈ gemeinsam mit R₉ und/oder R₉ gemeinsam mit R₁₀ jeweils mit C₁ und C₂ oder mit C₂ und C₃ oder mit C₃ und C₄ oder mit C₄, C₄ₐ und C₅ oder mit C₇ und C₈ oder mit C₈ und C₉ oder mit C₉ und C₁₀ einen aromatischen oder aliphatischen Ring mit 5 bis 10 Kohlenstoffatomen, wobei der Ring gegebenenfalls substituiert ist, insbesondere mit einem Halogen und/oder einer Alkyl-, Alkoxy-, Carbonyl-, Oxycarbonyl- oder Estergruppe und/oder mit einem aromatischen oder aliphatischen Ring, wobei diese Gruppen oder Ringe wie oben definiert sind, oder
• wenn R₃ und R₄ nicht die verschiedenen oben genannten Atome oder Gruppen oder Ringe repräsentieren, dann bildet R₃ gemeinsam mit R₄ eine Indolgruppe mit der Formel 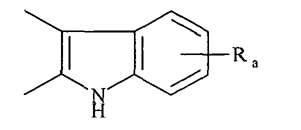
worin Rₐ wie oben definiert ist,
- Y repräsentiert:
• eine Gruppe -OR_{d}, wobei R_{d} ein Wasserstoffatom oder eine Alkyl-, Carbonyl-, Oxycarbonyl- oder Estergruppe, linear oder verzweigt, wobei diese Gruppen wie oben definiert sind, oder einen aromatischen oder aliphatischen Ring, wobei diese Ringe wie oben definiert sind, darstellt, oder
• eine Gruppe -NRₑR_{f}, wobei Rₑ und R_{f} unabhängig voneinander ein Wasserstoffatom, eine Alkyl-, Alkoxy-, Carbonyl-, Oxycarbonyl- oder Estergruppe, linear oder verzweigt, wobei diese Gruppen wie oben definiert sind, oder einen aromatischen oder aliphatischen Ring, wobei diese Ringe wie oben definiert sind, darstellen,
• oder eine Gruppe -SH,
• mit der Maßgabe dass, wenn R_{d} oder mindestens eines von Rₑ oder R_{f} nicht eines der verschiedenen oben genannten Atome oder eine(n) der oben genannten Gruppen oder Ringe darstellen, R_{d} oder mindestens eines von Rₑ oder R_{f} gemeinsam mit R₅ oder gemeinsam mit R₇ jeweils mit C₅ und C₆ oder mit C₆, C₆ₐ und C₇ einen aromatischen oder aliphatischen Heterocyclus mit 5 bis 10 Kohlenstoffatomen bildet, gegebenenfalls substituiert, insbesondere mit Halogen und/oder einer Alkyl-, Alkoxy-, Carbonyl-, Oxycarbonyl- oder Estergruppe und/oder einem aromatischen oder aliphatischen Ring, wobei diese Gruppen oder Ringe wie oben definiert sind,
- X ein Atom in anionischer Form, wie ein Halogenatom, insbesondere ein Brom- oder Chloratom, oder eine Gruppe von Atomen in anionischer Form, wie ein Perchlorat, repräsentiert und der Stickstoff des Heterocyclus A der Formel (I) in quartärer Form vorliegt und einerseits durch kovalente Bindung an den Kohlenstoff in der Position 11 und andererseits durch Ionenbindung an das oben definierte X gebunden ist, mit der Maßgabe, dass, wenn R₁ und R₁₀ nicht eines der verschiedenen oben genannten Atome oder eine(n) der oben genannten Gruppen oder Ringe repräsentieren, R₁ gemeinsam mit R₁₀ mit C₁, dem Stickstoff des Heterocyclus A der Formel (I), C₁₁ und C₁₀ einen aromatischen oder aliphatischen Heterocyclus mit 5 bis 10 Kohlenstoffatomen bildet, gegebenenfalls substituiert, insbesondere mit einem Halogen und/oder einer Alkyl-, Alkoxy-, Carbonyl-, Oxycarbonyl- oder Estergruppe und/oder einem aromatischen oder aliphatischen Ring, wobei diese Gruppen oder Ringe wie oben definiert sind,
zur Herstellung von Medikamenten zur Behandlung von Krankheiten, die mit einer Konstriktion der glatten Muskelzellen in den Geweben einhergehen, wie Krankheiten, die mit den Phänomenen der Vasokonstriktion im Rahmen von Gefäßerkrankungen in Verbindung stehen, insbesondere arterieller Hypertonie, oder Krankheiten, die mit den Phänomenen der Bronchokonstriktion im Rahmen von Atemwegserkrankungen in Verbindung stehen, insbesondere Asthma.

2. Verwendung nach Anspruch 1 von Benzo[c]chinoliziniiun-Derivaten der folgenden Formel (Ia): 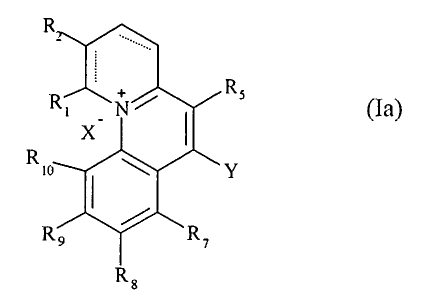 worin:
- R₁ und R₂ ein Wasserstoffatom repräsentieren oder gemeinsam mit C₁ und C₂ einen aromatischen Ring mit 6 Kohlenstoffatomen bilden,
- R₅ ein Wasserstoffatom oder eine lineare oder substituierte Alkylgruppe mit 1 bis 10 Kohlenstoffatomen, insbesondere eine Butylgruppe, oder einen Ester mit der Formel COOR', worin R' eine lineare oder substituierte Alkylgruppe mit 1 bis 10 Kohlenstoffatomen, insbesondere eine Ethylgruppe, darstellt, repräsentiert,
- Y eine Gruppe -OH, -SH, -NH₂ oder -NHCOCH₃ repräsentiert,
- R₇, R₈, R₉ und R₁₀ ein Wasserstoffatom repräsentieren oder mindestens eines von R₇, R₈, R₉ oder R₁₀ ein Halogenatom, insbesondere ein Chlor-, Brom- oder Fluoratom repräsentiert,
- X ein Halogenatom in anionischer Form, insbesondere ein Bromatom Br⁻ oder ein Chloratom Cl⁻, oder eine Gruppe von Atomen in anionischer Form repräsentiert.

3. Verwendung nach Anspruch 1 oder 2 von Benzo[c]chinolizinium-Derivaten der Formel (Ia), worin Y eine Gruppe -NH₂ oder -NHCOCH₃ darstellt.

4. Verwendung nach Anspruch 3 von folgenden Benzo[c]chinolizinium-Derivaten der Formel (Ia):

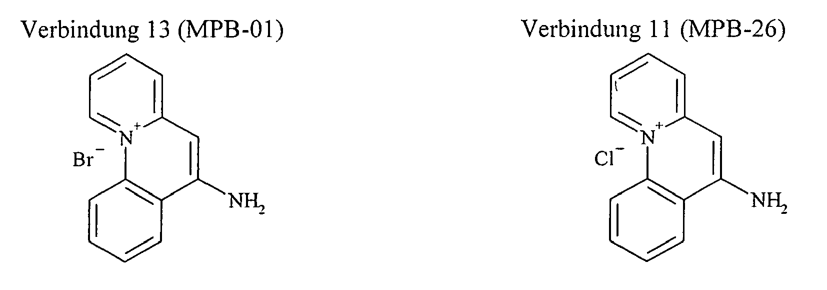

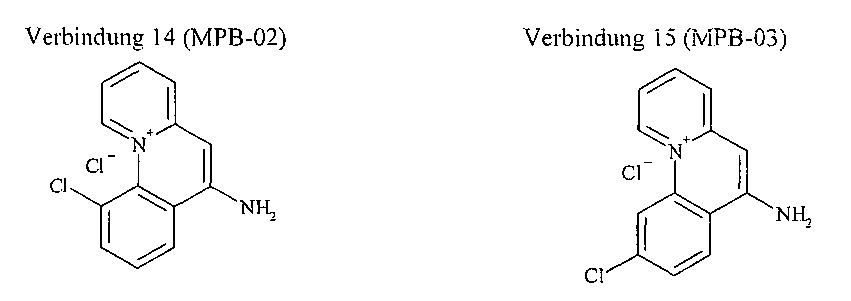

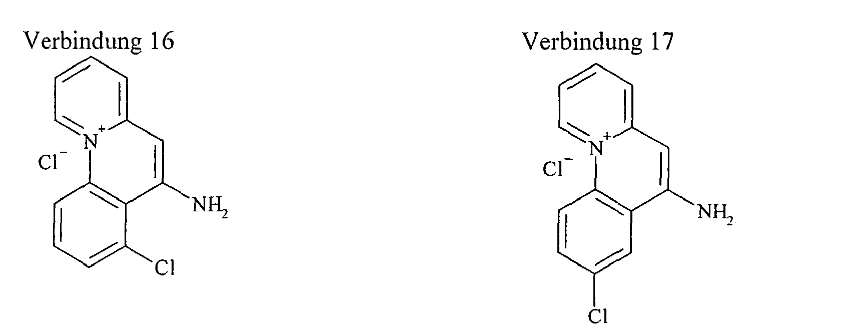

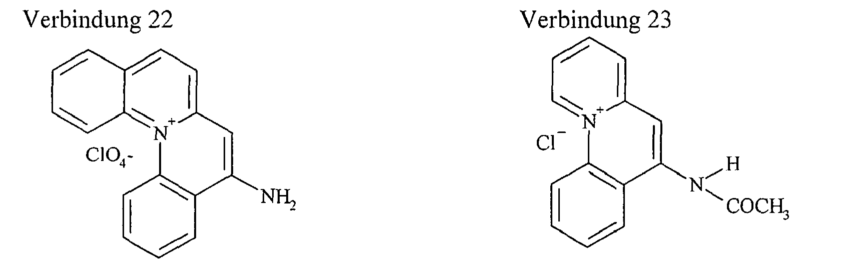

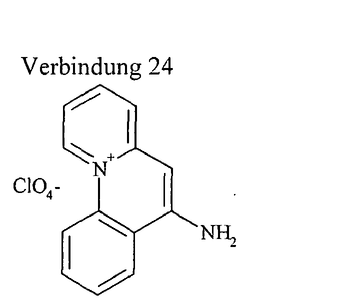

5. Verwendung nach Anspruch 1 oder 2 von Benzo[c]chinolizinium-Derivaten der Formel (la), worin Y für OH steht.

6. Verwendung nach Anspruch 5 von Benzo[c]chinolizinium-Derivaten der Formel (Ia), ausgewählt aus folgenden:

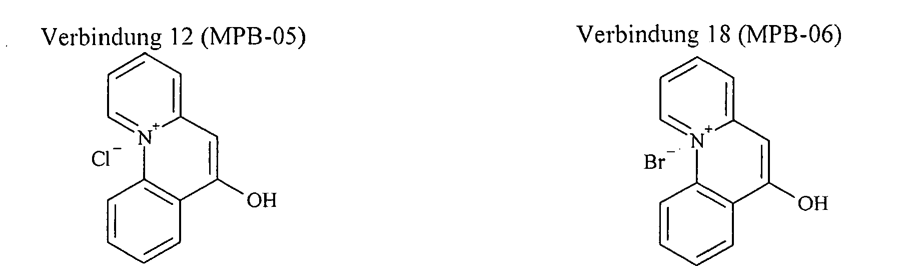

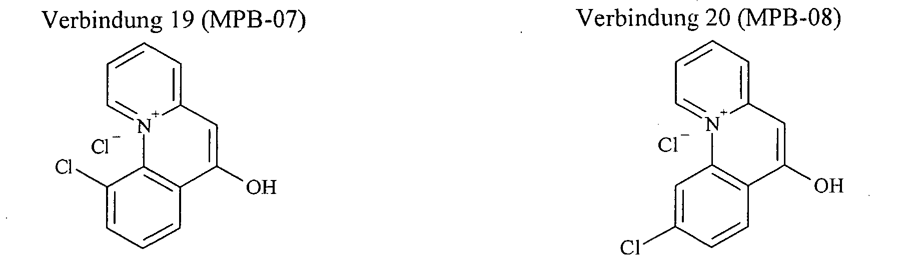

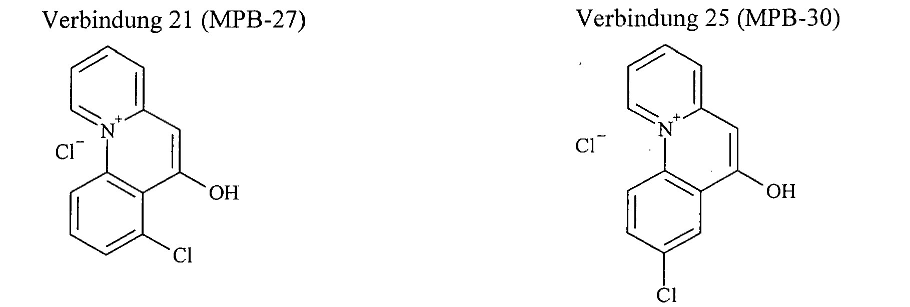

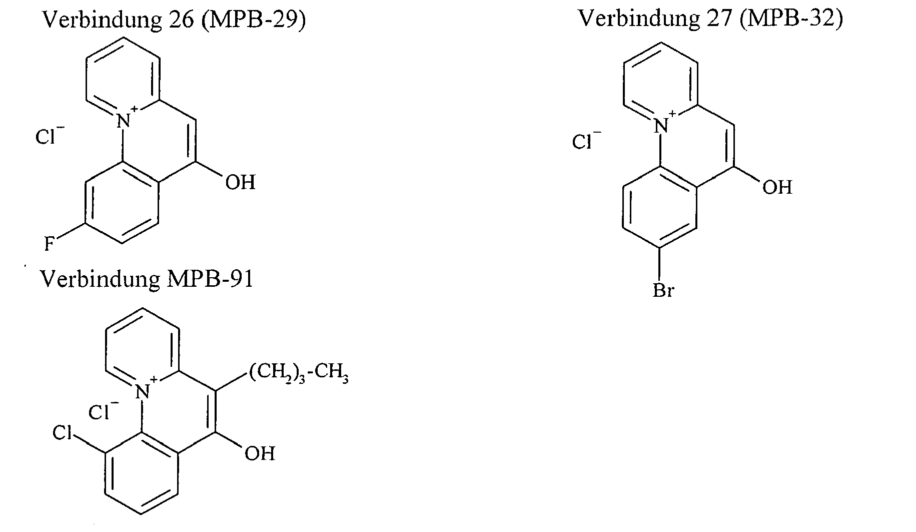

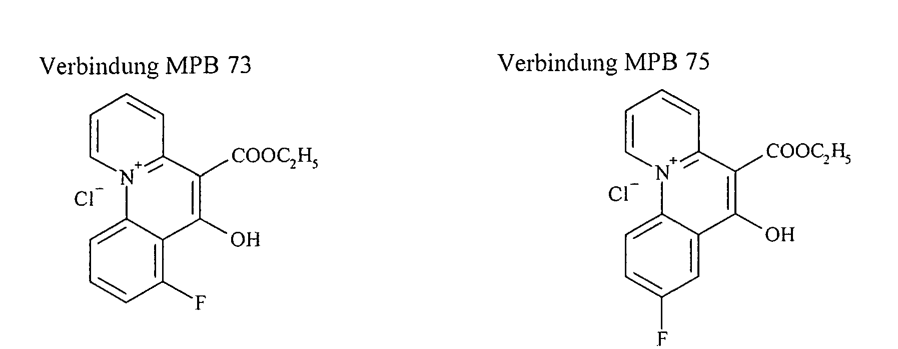

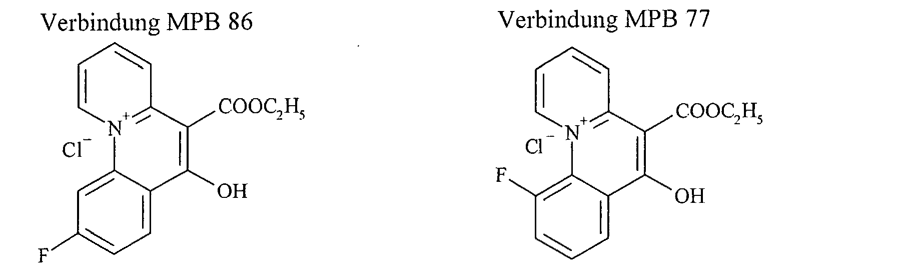

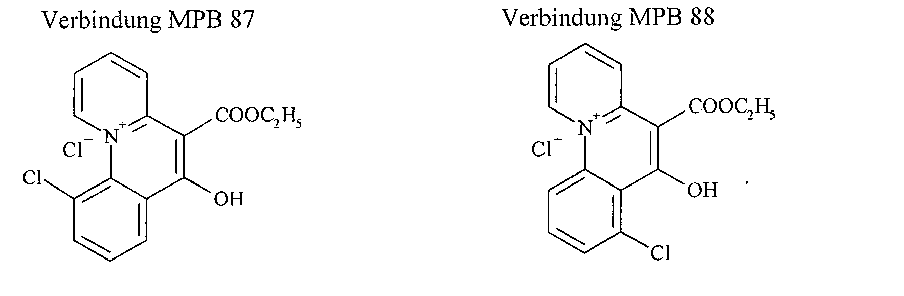

7. Verwendung nach Anspruch 1 oder 2 von Benzo[c]chinolizinium-Derivaten der Formel (Ia), worin Y für SH steht.

8. Verwendung nach Anspruch 7 von Benzo[c]chinolizinium-Derivaten der Formel (Ia), ausgewählt aus folgenden:

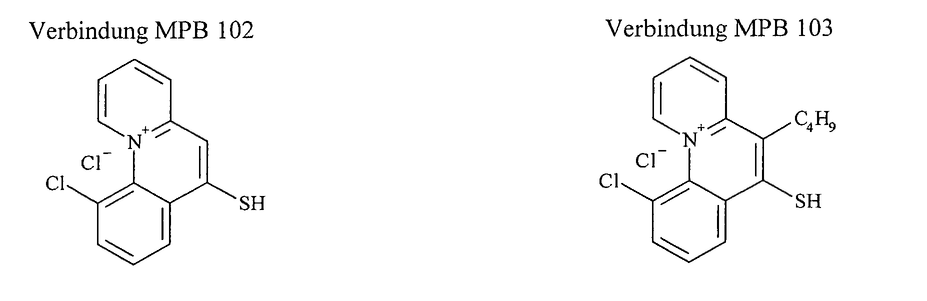

9. Verwendung nach Anspruch 5 von Derivaten der folgenden allgemeinen Formel (Ia-1): 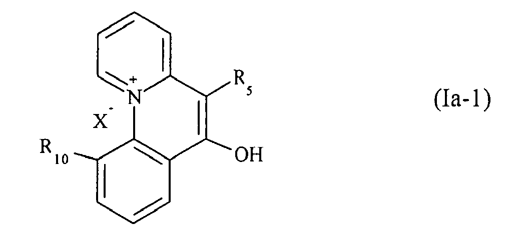 worin:
- R₅ ein Wasserstoffatom oder eine lineare oder substituierte Alkylgruppe mit 1 bis 10 Kohlenstoffatomen, insbesondere eine Butylgruppe, repräsentiert,
- R₁₀ ein Halogenatom, insbesondere ein Chlor-, Brom- oder Fluoratom, repräsentiert,
- X ein Halogenatom in anionischer Form, insbesondere ein Bromatom Br⁻ oder ein Chloratom Cl⁻, oder eine Gruppe von Atomen in anionischer Form, insbesondere Perchlorat ClO₄⁻, repräsentiert.

10. Verwendung nach Anspruch 9 des Derivats MPB-07 mit der folgenden Formel:

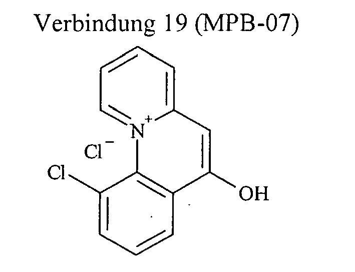

11. Verwendung nach Anspruch 9 des Derivats MPB-91 mit der folgenden Formel:

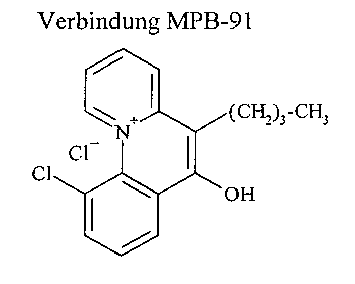

12. Verwendung nach Anspruch 1 von Derivaten mit der folgenden allgemeinen Formel (Ib): 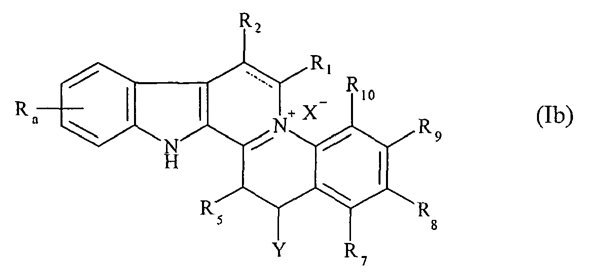 worin Rₐ, R₁, R₂, R₅, R₇, R₈, R₉, R₁₀, X und Y wie im Anspruch 1 definiert sind, und insbesondere von Verbindungen der Formel (Ib), worin:
- Rₐ ein Wasserstoffatom repräsentiert,
- R₁ und R₂ ein Wasserstoffatom repräsentieren und es keine Doppelbindung zwischen den beiden Kohlenstoffatomen gibt, die R₁ und R₂ tragen,
- R₅ ein Wasserstoffatom repräsentiert,
- R₇, R₈, R₉ und R₁₀ ein Wasserstoffatom repräsentieren oder eines von R₇, R₈, R₉ oder R₁₀ ein Halogenatom, insbesondere ein Chlor-, Brom- oder Fluoratom, repräsentiert,
- Y für -NH₂ oder -OH steht,
- X ein Halogenatom, insbesondere ein Brom- oder Chlor- oder Fluoratom, repräsentiert.

13. Verwendung nach Anspruch 10 von Derivaten der folgenden Formel (Ib-1): 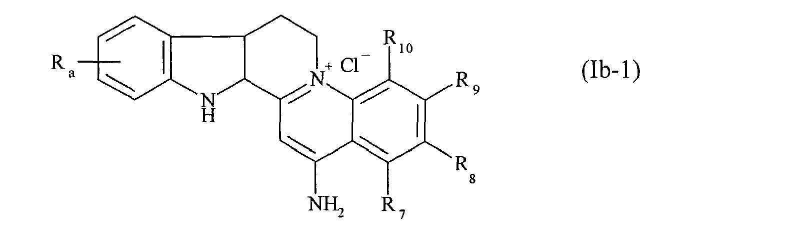 und spezieller der folgenden Verbindungen der Formel (Ib-1):
- Verbindung G: R₇ = Cl, R₈ = R₉ = R₁₀ = H,
- Verbindung H: R₇ = R₈ = R₉ = R₁₀ = H,
- Verbindung I: R₈ = Cl, R₇ = R₉ = R₁₀ = H,
- Verbindung J: R₉ = Cl, R₇ = R₈ = R₁₀ = H,
- Verbindung K: R₁₀ = Cl, R₇ = R₈ = R₉ = H,
- Verbindung L: R₉ = Br, R₇ = R₈ = R₁₀ = H.

14. Verbindungen der Formel (I), wie im Anspruch 1 definiert, worin R₅ einen Ester mit der Formel COOR' repräsentiert, worin R' eine lineare oder substituierte Alkylgruppe mit 1 bis 10 Kohlenstoffatomen, insbesondere eine Ethylgruppe, darstellt.

15. Verbindungen nach Anspruch 14 mit der Formel (Ia), wie im Anspruch 2 definiert, worin:
- R₁ und R₂ ein Wasserstoffatom repräsentieren oder gemeinsam mit C₁ und C₂ einen aromatischen Ring mit 6 Kohlenstoffatomen bilden,
- R₅ einen Ester mit der Formel COOR' repräsentiert, worin R' eine lineare oder substituierte Alkylgruppe mit 1 bis 10 Kohlenstoffatomen, insbesondere eine Ethylgruppe, darstellt,
- Y eine Gruppe -OH, -SH, -NH₂ oder -NHCOCH₃ repräsentiert,
- R₇, R₈, R₉ und R₁₀ ein Wasserstoffatom repräsentieren oder mindestens eines von R₇, R₈, R₉ oder R₁₀ ein Halogenatom, insbesondere ein Chlor-, Brom- oder Fluoratom, repräsentiert,
- X ein Halogenatom in anionischer Form, insbesondere ein Bromatom Br⁻ oder Chloratom Cl⁻, oder eine Gruppe von Atomen in anionischer Form repräsentiert.

16. Verbindungen nach Anspruch 14 oder 15 mit der Formel (Ia), worin R₅ einen Ester mit der Formel COOR' repräsentiert, worin R' eine lineare oder substituierte Alkylgruppe mit 1 bis 10 Kohlenstoffatomen, insbesondere eine Ethylgruppe, darstellt und Y eine Gruppe -OH repräsentiert.

17. Verbindungen nach einem der Ansprüche 14 bis 16 mit den folgenden Formeln:

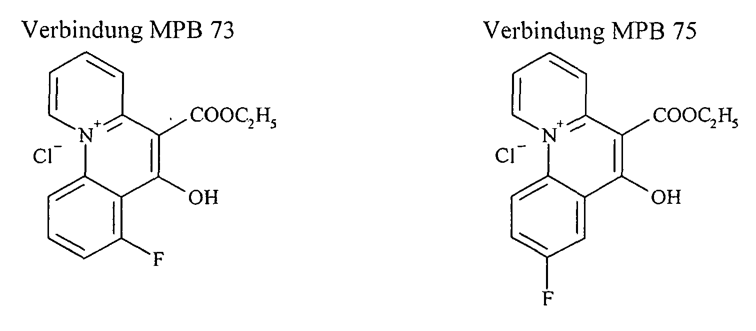

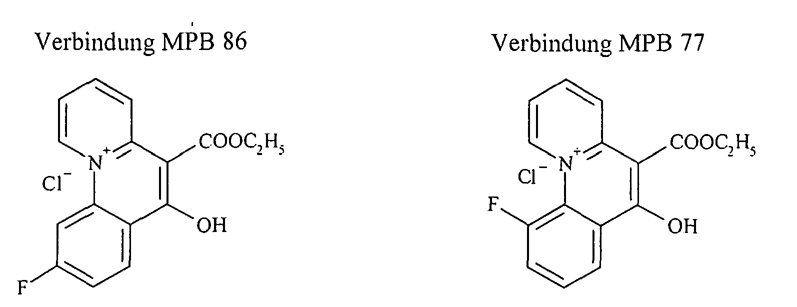

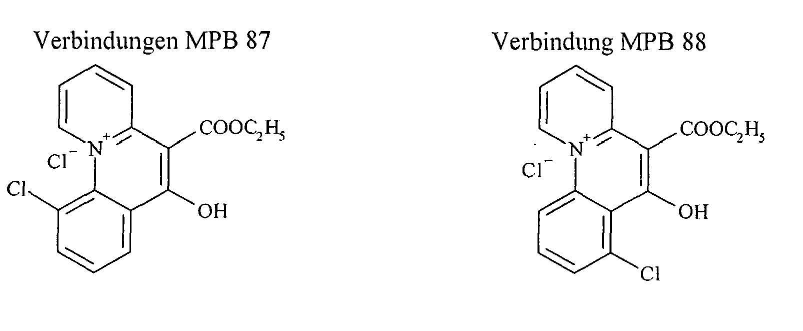

18. Verbindungen der Formel (I), wie im Anspruch 1 definiert, worin Y für SH steht.

19. Verbindungen nach Anspruch 18 mit der Formel (Ia), wie im Anspruch 2 definiert, worin:
- R₁ und R₂ ein Wasserstoffatom repräsentieren oder gemeinsam mit C₁ und C₂ einen aromatischen Ring mit 6 Kohlenstoffatomen bilden,
- R₅ ein Wasserstoffatom oder eine lineare oder substituierte Alkylgruppe mit 1 bis 10 Kohlenstoffatomen, insbesondere eine Butylgruppe, repräsentiert,
- Y für eine Gruppe SH steht,
- R₇, R₈, R₉ und R₁₀ ein Wasserstoffatom repräsentieren oder mindestens eines von R₇, R₈, R₉ oder R₁₀ ein Halogenatom, insbesondere ein Chlor-, Brom- oder Fluoratom, repräsentiert,
- X ein Halogenatom in anionischer Form, insbesondere ein Bromatom Br⁻ oder ein Chloratom Cl⁻, oder eine Gruppe von Atomen in anionischer Form repräsentiert.

20. Verbindungen nach Anspruch 18 oder 19 mit den folgenden Formeln:

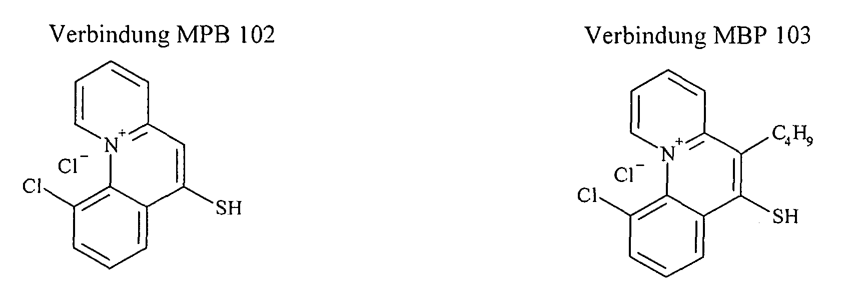

21. Pharmazeutische Zusammensetzungen, die mindestens eine Verbindung wie in einem der Ansprüche 14 bis 20 definiert zusammen mit einem pharmazeutisch unbedenklichen Träger aufweisen.
